(19)

**Europäisches Patentamt**
**European Patent Office**
**Office européen des brevets**

(11) **EP 4 184 520 A1**

(12) **EUROPEAN PATENT APPLICATION**

(43) Date of publication:
**24.05.2023 Bulletin 2023/21**

(51) International Patent Classification (IPC):
***G16H 20/40*** *(2018.01)*

(21) Application number: **21306604.6**

(52) Cooperative Patent Classification (CPC):
**G16H 20/40**

(22) Date of filing: **18.11.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Gambro Lundia AB**
**226 43 Lund (SE)**

(72) Inventors:
- **POUCHOULIN, Dominique**
  **01390 TRAMOYES (FR)**
- **HERTZ, Thomas**
  **22732 LUND (SE)**

(74) Representative: **Ponzellini, Gian-Marco**
**PGA S.p.A., Milano**
**Succursale di Lugano**
**Via Castagnola 21C**
**6900 Lugano (CH)**

(54) **PLASMA ELECTROLYTE MANAGEMENT SYSTEM, METHODS, AND APPARATUS FOR CONTINUOUS RENAL REPLACEMENT THERAPIES (RRT)**

(57) Plasma electrolyte management system, methods, and apparatus for continuous renal replacement therapies (RRT) are disclosed. The example system, methods, and apparatus use one or more kinetic physiological models to calculate a current electrolyte rate of change in plasma sodium or other electrolytes based on current data for patient weight, input/output of water, sodium, and potassium (e.g., input/output of known infusions, dialysis, urine, blood loss, etc.) This input/output data is acquired through known data, such as infusion data, dialysis data, urine data, and blood loss data, which are typically stored to a patient's electronic medical record ("EMR") as the data is generated/received. The use of available point-in-time input/output data to generate accurate electrolyte concentration estimations means that fewer (or none) blood tests are needed, thereby providing accurate electrolyte determinations without frequent burdensome blood analyses.

PROCESSOR (40) CLINICIAN SERVER (106)

START

502 — RECEIVE PATIENT DATA

504 — PREDICT FLUID, WATER, ELECTROLYTE INPUTS/OUTPUTS

506 — APPLY A KINETIC PHYSIOLOGICAL MODEL TO THE PREDICTED FLUID, WATER, ELECTROLYTE INPUTS/OUTPUTS

508 — OUTPUT A CURRENT AND/OR FURTHER PLASMA ELECTROLYTE CONCENTRATION

510 — DETERMINE A RATE OF CHANGE

512 — IS CORRECTION NEEDED? NO

YES

514 — DETERMINE A RATE OF CORRECTION

516 — APPLY THE RATE OF CORRECTION

FIG. 5

EP 4 184 520 A1

**Description**

BACKGROUND

Due to various causes, a person's renal system can fail. Renal failure produces several physiological derangements. For instance, it is no longer possible for a person with renal failure to balance water and minerals or to excrete daily metabolic load. Additionally, toxic end products of metabolism, such as, urea, creatinine, uric acid and others, may accumulate in a patient's blood and tissue.

Reduced kidney function and, above all, kidney failure is treated with dialysis or renal replacement therapies ("RRT"). Dialysis and RRT remove waste, toxins and excess water from the body that normal functioning kidneys would otherwise remove. Dialysis treatment and RRT for the replacement of kidney function is critical to many people because the treatment is lifesaving.

One type of kidney failure therapy or RRT is Hemodialysis ("HD"), which in general uses diffusion to remove waste products from a patient's blood. A diffusive gradient occurs across a semi-permeable dialyzer between the blood and an electrolyte solution, called dialysate or dialysis fluid, to cause diffusion. The diffusion occurs externally from the patient, where an extracorporeal circuit is used to remove uncleansed blood and return cleansed blood to the patient.

Hemofiltration ("HF") is an alternative renal replacement therapy that relies on a convective transport of toxins from a patient's blood. HF is accomplished by adding substitution or replacement fluid to the extracorporeal circuit during treatment. The substitution fluid and the fluid accumulated by the patient in between treatments is ultrafiltered over the course of the HF treatment using a dialyzer or other filter, thereby providing a convective transport mechanism that is particularly beneficial in removing middle and large toxic molecules.

Hemodiafiltration ("HDF") is a treatment modality that combines convective and diffusive clearances. HDF uses dialysis fluid flowing through a dialyzer, similar to standard hemodialysis, to provide diffusive clearance. In addition, substitution solution is provided directly to the extracorporeal circuit, providing convective clearance.

Acute patients are generally prescribed continuous renal replacement therapy ("CRRT") treatments in which filtering and fluid removal occurs more slowly throughout the day. It is not uncommon for intensive care patients to have severe electrolyte imbalances when starting on CRRT. Correction of severe electrolyte imbalances may have to be handled at different paces. For example, severe hyperkalemia typically requires fast intervention and correction. In another example, severe dysnatremia should not be corrected too fast as to prevent, for example, brain damage.

There are known kinetics physiological models that calculate a net balance and change of electrolyte concentration(s) in the body of a patient, as represented by a plasma electrolyte concentration. These known models require an input value of an electrolyte plasma concentration and patient weight data as a starting point. These input values are typically obtained through blood sampling.

The known kinetics models have four main components including a physiological model, a net balance model, overall fluid input/output data, and data related to electrolyte gains/losses. The physiological model represents a distribution of electrolytes in a patient's body. In the case of sodium, this may be a single pool model of extracellular water, or a more complex model including intracellular volume, potassium concentrations, and sodium stores in bones and skin. Overall, the model takes into account volume changes that occur during a RRT. The net balance model calculates a net balance of the electrolyte from an RRT therapy. This model integrates primarily a clearance model of a hemodialyzer/filter as a function of operating flow rates for blood, dialysate, and/or filtration and electrolyte concentration in the fluids. Computation of the mass transfer rate of electrolyte to the effluent requires a computation of electrolyte plasma water concentration at a filter inlet, as well as consideration of the Donnan effect when dealing with charged species.

The overall fluid input/output data enables a determination of measurable changes to the electrolyte distribution volume. The data includes information indicative of infusion fluids, blood transfusions, urine output, bleeding, breathing, or sweating. The data may be obtained or received directly from an infusion pumps, urine collecting device, model estimates (breathing, sweating), or via manual input by nurses. The data related to electrolyte gains/losses enables a computation of net electrolyte balance outside an RRT therapy. Electrolyte losses in stools may also be considered.

An issue with known models is that they are not readily available to clinicians. For instance, clinicians do not have access to software that analyzes patient fluid intake and plasma electrolyte concentrations to determine trends and treatment recommendations. Instead, many clinicians rely on standardized treatment guidelines to minimize risks and perform tight monitoring of a patient.

In addition to availability issues, known models are only academic and do not provide clinical decision support. Instead, known models determine a net balance and change of electrolyte concentration(s) in the body of a patient. It is then left to the clinician to determine whether parameters of a CRRT for an acute patient should be changed to balance electrolyte concentration(s), and if so, how parameters should be changed while not changing the electrolyte concentration(s) too fast or risk causing adverse patient conditions, such as high blood pressure, low blood pressure, and/or serious neurologic complications.

SUMMARY

A plasma electrolyte management system, methods, and apparatus for (continuous) RRTs are disclosed herein. The example system, methods, and apparatus use one or more kinetic physiological models to calculate a current electrolyte rate of change in plasma sodium or other electrolytes based on current data for patient weight, fluid status, input/output of water, sodium, and potassium (e.g., input/output of known infusions, dialysis, urine, blood loss, etc.) This input/output data is acquired through known data, such as infusion data, dialysis data, urine data, and blood loss data, which are typically stored to a patient's electronic medical record ("EMR") as the data is generated/received. The use of available point-in-time input/output data to generate accurate electrolyte concentration estimations means that fewer (or none) blood tests are needed, thereby providing accurate electrolyte determinations without frequent burdensome blood analyses.

The example plasma electrolyte management system, methods, and apparatus may also be configured to use one or more kinetic physiological models to predict future plasma sodium (or other electrolyte) concentrations based on current readily available (e.g., streamlined) data for input/output of water, sodium, and potassium. The calculated electrolyte rate of change may be provided to clinicians as current/predicted rates of change for a period of interest (e.g., a prediction time window), such as a next 24 hours, 48 hours, 96 hours, etc. The prediction from available single time point measurements is conducted without having to perform multiple blood tests. Further, since the available input/output data may be received in real-time or near real-time, the electrolyte concentration predictions may also be provided in real-time or new real-time for clinicians instead of having to wait hours for a blood test to be processed.

In some embodiments, the example plasma electrolyte management system, methods, and apparatus may be configured to analyze intermediate plasma sodium measurements (or other plasma electrolyte measurements) in conjunction with one or more prescribed RRT or dialysis treatments to verify treatment is progressing as predicted and/or prescribed. For example, the example plasma electrolyte management system, methods, and apparatus may compare a trend of plasma electrolyte measurements against predicted plasma electrolyte concentrations to determine if a significant deviation is present. When a deviation is present, the example plasma electrolyte management system, methods, and apparatus may be implemented may generate an alert or alarm for a clinician.

The example plasma electrolyte management system, methods, and apparatus are configured to calculate RRT parameters such as a target/required dialysate or plasma sodium concentration, a target/required dialysate or plasma potassium concentration, a target/required flow rate, etc. The RRT parameters are calculated to meet, for example, a specific prescribed plasma sodium target (e.g., achieving plasma sodium concentration at a given time T, while keeping plasma sodium rate of change below safe limit, such as X mmol/L/24h). The rate of change for sodium is especially important for patients.

The example plasma electrolyte management system, methods, and apparatus may determine, for example, adjustments of RRT parameters to achieve a prescribed plasma sodium correction profile. The plasma electrolyte management system, methods, and apparatus may automatically adjust an RRT machine based on the adjusted parameters. In some instances, a CRRT machine may include an embedded kinetic physiological model for one or more electrolytes. The model is configured to provide for automatic control of the CRRT machine to achieve a target equilibrium concentration and/or a pace/rate of correction through adjustment of RRT or CRRT parameters. Such a configuration provides local open loop control based on one or more physiological models.

In other embodiments, the kinetic physiological model is located in a distributed computing environment or at a server. In these examples, the distributed computing environment or the server is configured to receive input/output data from a CRRT machine, an infusion pump, etc. and determine the parameter adjustments for achieving a target equilibrium concentration and/or a pace/rate of correction. The distributed computing environment or the server provides the recommended parameter adjustments to a user interface of a clinician device for confirmation. After confirmation is received, the distributed computing environment or the server transmits the parameter adjustments (as a modified prescription) to the CRRT machine. In instances where a pace/rate of correction changes overtime, the model may output a rate for a desired time period to the CRRT machine, then later output a second rate. The distributed computing environment or the server may use additional input/output data to confirm a patient's electrolyte concentrations are changing as predicted before providing instructions to change to the second rate.

In light of the disclosure set forth herein, and without limiting the disclosure in any way, in a first aspect of the present disclosure, which may be combined with any other aspect, or portion thereof, described herein a system for plasma electrolyte management for continuous renal replacement therapies ("RRT") includes a first memory device storing patient data for a patient. The patient data includes patient blood data from an initial blood test, fluid status, body weight, infusion data, dialysis data, urine output, and RRT prescription parameters associated with a prescribed clinical target. The system also includes a second memory device storing a kinetic physiological model configured to calculate a current and estimated future plasma electrolyte concentration in the patient using the patient data. The system further includes a processor communicatively coupled to the first memory and the second memory. The processor is configured to determine or receive a current plasma electrolyte concentration, calculate a electrolyte rate of change and an estimated future plasma electrolyte concentration in the patient using the kinetic physiological model taking into account the current plasma electrolyte concentration, and cause the electrolyte rate of change to be displayed.

In accordance with a second aspect of the present disclosure, which may be used in combination with any other aspect listed herein unless stated otherwise, the processor is further configured to compare the electrolyte rate of change to a threshold, and when the electrolyte rate of change exceeds the threshold, generate a message indicative that at least some of the RRT prescription parameters should be changed to reduce the electrolyte rate of change or achieve the clinical target.

In accordance with a third aspect of the present disclosure, which may be used in combination with any other aspect listed herein unless stated otherwise, the processor, the first memory device, and the second memory device are located in a RRT machine.

In accordance with a fourth aspect of the present disclosure, which may be used in combination with any other aspect listed herein unless stated otherwise, the RRT machine includes at least one of a continuous RRT machine or a hemodialysis machine.

In accordance with a fifth aspect of the present disclosure, which may be used in combination with any other aspect listed herein unless stated otherwise, the processor is remote from and in communication with a RRT machine.

In accordance with a sixth aspect of the present disclosure, which may be used in combination with any other aspect listed herein unless stated otherwise, the processor is further configured to calculate a change to at least some of the RRT prescription parameters to reduce the electrolyte rate of change to be below the threshold, and apply the calculated change to the RRT prescription parameters such that the RRT machine operates according to the changed RRT prescription parameters, or cause the calculated change to the RRT prescription parameters to be displayed for clinician verification.

In accordance with a seventh aspect of the present disclosure, which may be used in combination with any other aspect listed herein unless stated otherwise, the calculated change to the at least some of the RRT prescription parameters is configured to achieve at least one of a target equilibrium concentration of the plasma electrolyte concentration or a pace/rate of correction of the plasma electrolyte concentration.

In accordance with an eighth aspect of the present disclosure, which may be used in combination with any other aspect listed herein unless stated otherwise, the RRT prescription parameters includes first RRT prescription parameters corresponding to a first pace/rate of correction and second RRT prescription parameters corresponding to a second pace/rate of correction, and the processor is further configured to apply the first RRT prescription parameters to the RRT machine at a first time and apply a second calculated change to the second RRT prescription parameters to the RRT machine at a second time after the first time.

In accordance with a ninth aspect of the present disclosure, which may be used in combination with any other aspect listed herein unless stated otherwise, the processor is further configured to receive additional patient data generated at any time, use the kinetic physiological model to determine a new current plasma electrolyte concentration and a new estimated future plasma electrolyte concentration in the patient for calculating an instantaneous, new electrolyte rate of change, and determine new RRT prescription parameters based on the new electrolyte rate of change and the new current plasma electrolyte concentration.

In accordance with a tenth aspect of the present disclosure, which may be used in combination with any other aspect listed herein unless stated otherwise, the processor is located in a server or a distributed computing environment, and wherein the first memory device and the second memory device are located at a centralized database in communication with the sever or located within the distributed computing environment.

In accordance with an eleventh aspect of the present disclosure, which may be used in combination with any other aspect listed herein unless stated otherwise, the plasma electrolyte includes at least one of sodium, potassium, or phosphorus.

In accordance with a twelfth aspect of the present disclosure, which may be used in combination with any other aspect listed herein unless stated otherwise, the estimated future plasma electrolyte concentration is calculated for a subsequent time period of at least one of four hours, eight hours, twelve hours, sixteen hours, 24 hours, 48 hours, or 96 hours.

In accordance with a thirteenth aspect of the present disclosure, which may be used in combination with any other aspect listed herein unless stated otherwise, the RRT prescription parameters includes at least one of patient fluid removal rate, Qeff effluent flow rate, a filter K0.A, a blood flow rate, a pre-replacement fluid flow rate, a dialysate or dialysis fluid flow rate, a post-replacement fluid flow rate, a fluid removal rate, a pre-replacement electrolyte concentration, a post-replacement electrolyte concentration, or a dialysate electrolyte concentration.

In accordance with a fourteenth aspect of the present disclosure, which may be used in combination with any other aspect listed herein unless stated otherwise, the processor is further configured to estimate the future plasma electrolyte concentration in the patient by using the infusion data, dialysis data, urine output, and RRT prescription parameters to estimate future water, fluid, and electrolyte input/output amounts, and applying the estimated future water, fluid, and electrolyte input/output amounts to the kinetic physiological model.

In accordance with a fifteenth aspect of the present disclosure, which may be used in combination with any other aspect listed herein unless stated otherwise, the dialysis data includes electrolyte removal in effluent determined by the processor computing the current plasma electrolyte concentration using a RRT machine that is fluidly coupled to the patient.

In accordance with a sixteenth aspect of the present disclosure, which may be used in combination with any other aspect listed herein unless stated otherwise, the kinetic physiological model to determine the estimated future plasma electrolyte concentration in the patient includes periodic 5% to 20% per hour down times for changing bags of a RRT machine and a long down time of 30 to 180 minutes to replace an extracorporeal circuit of the RRT machine at least once during a prediction window.

In accordance with a seventeenth aspect of the present disclosure, which may be used in combination with any other aspect listed herein unless stated otherwise, the processor is further configured to receive an indication of a down time of a RRT machine, update the kinetic physiological model based on a length of the down time, and at least one of generate an alert message indicative of the down time, generate a message indicative of a new current plasma electrolyte concentration and a new estimated future plasma electrolyte concentration in the patient based on the down time, or generate a message indicative that the new RRT prescription parameters should be changed to reach the clinical target when the RRT is resumed.

In accordance with an eighteenth aspect of the present disclosure, which may be used in combination with any other aspect listed herein unless stated otherwise, the system is for acute RRTs.

In accordance with a nineteenth aspect of the present disclosure, which may be used in combination with any other aspect listed herein unless stated otherwise, a system for plasma electrolyte management for continuous renal replacement therapies ("RRT") includes a RRT machine configured to administer a RRT to a patient according to a RRT prescription parameters associated with a prescribed clinical target and a first memory device storing patient data for a patient. The patient data includes patient blood data from an initial blood test, infusion data, dialysis data, and urine output. The system also includes a second memory device storing a kinetic physiological model configured to calculate a current and estimated future plasma electrolyte concentration in the patient using the patient data. The system further includes a processor communicatively coupled to the RRT machine, the first memory, and the second memory. The processor is configured to use the kinetic physiological model to determine a current plasma electrolyte concentration and an estimated future plasma electrolyte concentration in the patient over a prediction window, compare the current plasma electrolyte concentration and the estimated future plasma electrolyte concentration to the prescribed clinical target, determine at least some new RRT prescription parameters based on the comparison to meet the prescribed clinical target, and transmit a message to the RRT machine with the new RRT prescription parameters, the message causing the RRT machine to administer the RRT using the new RRT prescription parameters.

In accordance with a twentieth aspect of the present disclosure, which may be used in combination with any other aspect listed herein unless stated otherwise, the processor, the first memory device, and the second memory device are located in a RRT machine.

In accordance with a twenty-first aspect of the present disclosure, which may be used in combination with any other aspect listed herein unless stated otherwise, the RRT machine includes at least one of a continuous RRT machine or a hemodialysis machine.

In accordance with a twenty-second aspect of the present disclosure, which may be used in combination with any other aspect listed herein unless stated otherwise, the processor is further configured to compare a predicted future plasma electrolyte concentration based on the new RRT prescription parameters to at least one threshold, when the predicted future plasma electrolyte concentration exceeds the at least one threshold, modify the RRT prescription parameters to be below the threshold.

In accordance with a twenty-third aspect of the present disclosure, which may be used in combination with any other aspect listed herein unless stated otherwise, the new the RRT prescription parameters are configured to achieve at least one of a target equilibrium concentration of the plasma electrolyte concentration or a pace/rate of correction of the plasma electrolyte concentration.

In accordance with a twenty-fourth aspect of the present disclosure, which may be used in combination with any other aspect listed herein unless stated otherwise, a system for plasma electrolyte management for continuous renal replacement therapies ("RRT") includes a first memory device storing patient data for a patient. The patient data includes patient blood data from an initial blood test, fluid status, infusion data, body weight, an expected/prescribed patient volume correction ("$V_{corr}$") over a prediction time period, and RRT prescription parameters associated with a prescribed clinical target. The system also includes a second memory device storing a kinetic physiological model configured to calculate a current and estimated future plasma electrolyte concentration in the patient using the patient data. The system further includes a processor communicatively coupled to the first memory and the second memory. The processor is configured to determine or receive a current plasma electrolyte concentration, calculate a correction rate of the patient fluid volume ("Qnet") over the prediction time period, calculate at least one of a electrolyte rate of change or an estimated future plasma electrolyte concentration in the patient using the kinetic physiological model taking into account the current plasma electrolyte concentration, and the correction rate of the patient fluid volume ("Qnet"), and cause the at least one of the electrolyte rate of change or the estimated future plasma electrolyte concentration to be displayed.

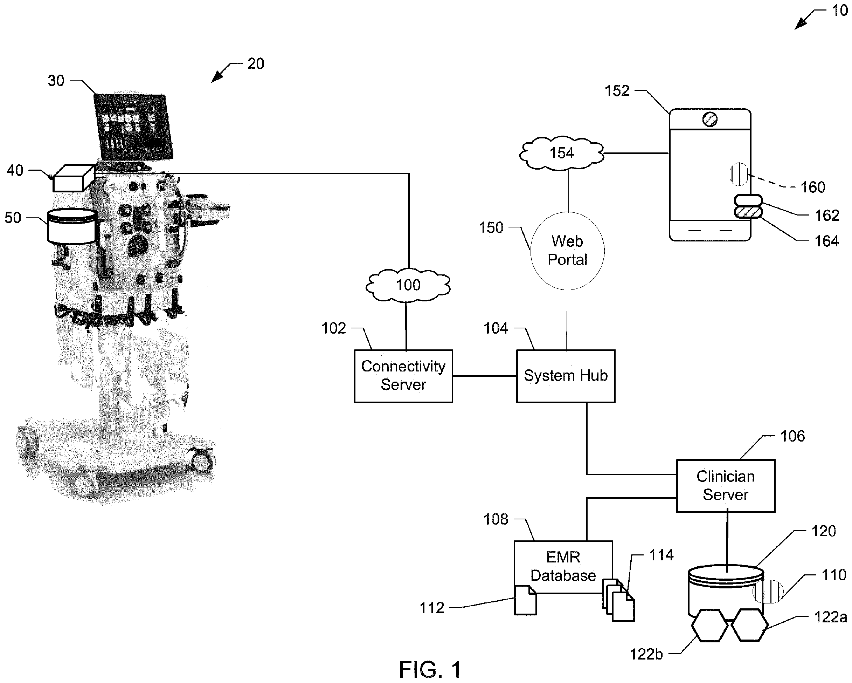

In a twenty-fifth aspect, any of the features, functionality and alternatives described in connection with any one or more of Figs. 1 to 15 may be combined with any of the features, functionality and alternatives described in connection with any other of Figs. 1 to 15.

In light of the present disclosure and the above aspects, it is therefore an advantage of the present disclosure to provide a prediction of an evolution over time of a patient plasma concentration for an electrolyte of interest without needing to conduct frequent blood tests.

It is another advantage of the present disclosure to determine and/or recommend RRT prescriptions that are predicted to deliver a defined patient outcome over time.

It is a further advantage of the present disclosure to automatically provide a RRT machine an adjusted RRT prescription that is predicted to achieve a desired patient outcome over time.

Additional features and advantages are described in, and will be apparent from, the following Detailed Description and the Figures. The features and advantages described herein are not all-inclusive and, in particular, many additional features and advantages will be apparent to one of ordinary skill in the art in view of the figures and description. Also, any particular embodiment does not have to have all of the advantages listed herein and it is expressly contemplated to claim individual advantageous embodiments separately. Moreover, it should be noted that the language used in the specification has been selected principally for readability and instructional purposes, and not to limit the scope of the inventive subject matter.

BRIEF DESCRIPTION OF THE FIGURES

Fig. 1 illustrates a diagram of a plasma electrolyte management system, according to an example embodiment of the present disclosure.

Fig. 2 illustrates a diagram of an extracorporeal circuit and a dialysate circuit of an RRT machine, according to an example embodiment of the present disclosure.

Fig. 3 illustrates another diagram of a plasma electrolyte management system, according to an example embodiment of the present disclosure.

Fig. 4 is a diagram illustrative of the modeling performed by the plasma electrolyte management system of Figs. 1 and 3, according to an example embodiment of the present disclosure.

Fig. 5 is a flow diagram of an example procedure to use a kinetic physiological model to estimate plasma electrolyte concentrations in a patient, according to an example embodiment of the present disclosure.

Fig. 6 is an example user interface that displays plasma electrolyte concentration information for a clinician, according to an example embodiment of the present disclosure.

Figs. 7 to 15 show example graphs of plasma electrolyte concentrations as determined by one or more kinetic physiological models disclosed herein, according to example embodiments of the present disclosure.

DETAILED DESCRIPTION

A plasma electrolyte management system, methods, and apparatus for continuous RRTs are disclosed herein. The example plasma electrolyte management system, methods, and apparatus are configured to use one or more kinetic physiological electrolyte models to provide clinical decision support for the management of plasma electrolytes for a patient undergoing dialysis or RRT for acute kidney conditions. The example plasma electrolyte management system, methods, and apparatus are especially appropriate for acute patients undergoing CRRT.

CRRT systems are configured for delivering very specific treatments designed for patients versing in acute states of illness and who have temporarily lost (partially or entirely) their kidney function. In this respect, CRRT systems may be structurally and/or operationally different from extracorporeal blood treatment systems designed for chronic patient care. In contrast to chronic patients, acute patients temporarily experience loss of their kidney function typically due to a contemporaneous state of severe injury or during recovery from surgery. Consequently, acute patients are often extremely weak and typically not in a condition to be submitted to regular dialysis treatment, which could further deteriorate their state and lead to serious and possibly life-threatening complications. Under circumstances as described, CRRT systems are designed to individually treat a patient exhibiting very poor health, without inducing further stress to the patient body, in particular without allowing vital parameters pertaining to the patient's blood to deviate from ideal or near-ideal values. Within the scope of this document CRRT systems are, thus, inherently characterized by one or more of the following features. CRRT involves renal replacement therapy, meaning an adjuvant therapy aimed firstly at facilitating continuous fluid removal in diuretic-resistant or acute renal failure patients. Therefore, CRRT systems inherently require a continuous net fluid removal from the patient. In other words, a CRRT system requires a fluid balance control system, such as a weight loss control system, configured to generate a continuous net weight loss rate (as opposed to merely controlling parameters to enable achieving a desired target weight loss as typically found in chronic patient care). Furthermore, acute patients experience extravascular fluid overload, which cannot be safely removed within a short period of time (e.g. within a few hours of chronic treatment) without causing potentially severe consequences (e.g. hypovolemic shock, arrhythmia, hypoxemia, hypoventilation, etc.). Therefore, a CRRT system must inherently include a much more accurate control over system parameters, in particular flow rates, in order to ensure that the required low flow rates of both blood circulating extra-corporeally and of treatment fluid (infused in the extracorporeal circuit or diffused through the dialyzer) are used. Moreover, CRRT treatment is performed continuously (e.g. for days or even weeks, without interruption/with minimal interruptions e.g., downtimes to change bags). Therefore, treatment settings in CRRT are based on flow rate settings, rather than settings pertaining to some specified treatment time (which would be unknown as acute patients may require treatment for an unknown time). Consequently, operation of CRRT systems cannot be based on some pre-defined absolute weight loss to be achieved, but rather on a meticulously controlled fluid balance in the patient, requiring continuous adjustments to a number of operating parameters, which have to be controlled and maintained during the entire (and a priori unknown) treatment time, based on a set weight-loss rate. Additionally, CRRT renal replacement therapy involves therapy substituting kidney functions for a relatively long time period and, thus, a CRRT system further requires at least either fresh dialysis liquid exchange in the dialyzer (in order to remove unwanted substances from blood and to add desired substances to the blood by diffusion) and/or fresh infusion fluid in combination with ultrafiltration (in order to remove unwanted substances from blood and to add desired substances to the blood by convection).

At least for the reasons set forth above, CRRT systems need to exhibit specific technical features enabling the system to:

- Allow setting of a patient net removal rate,
- Continuously remove excess water in accordance with a set patient net removal rate,
- Operate continuously at comparably low flow rates compatible with CRRT, and
- Balance ion equilibrium by means of proper dialysis being performed and/or by means of substitution fluid continuously being delivered at controlled flow rates and with adequate composition.

Finally, in order to ready a CRRT apparatus as soon as possible given the acute situation of a patient requiring a treatment and given the absence of advance notice of the emergency situation requiring treatment, the CRRT machine is dressed using an integrated disposable set, wherein all the lines and the filtration unit are grouped together and already properly connected in the disposable set.

The plasma electrolyte management system, methods, and apparatus may also be used for patients undergoing sustained low-efficiency dialysis ("SLED") and/or hemodialysis.

The disclosed plasma electrolyte management system, methods, and apparatus are discussed in the context of sodium plasma electrolyte. However, it should be appreciated that the plasma electrolyte management system, methods, and apparatus may be used for determining other plasma electrolytes such as potassium and phosphates, calcium, magnesium, chloride, bicarbonate, and/or sulfates. The plasma electrolyte management system, methods, and apparatus may use a separate kinetic physiological model for each plasma electrolyte of interest or one or more models configured to the determination of multiple plasma electrolytes.

The example plasma electrolyte management system, methods, and apparatus are configured to use readily available patient data (including fluid input/output data) to determine a current patient plasma concentration for the electrolyte of interest. The example plasma electrolyte management system, methods, and apparatus may also predict an evolution over time (e.g., four hours, eight hours, twelve hours, 24 hours, 48 hours, 96 hours, etc.) patient plasma concentration for the electrolyte of interest. In some instances, the plasma electrolyte management system, methods, and apparatus may also use known RRT parameters in the calculations. The RRT parameters may include fluid composition or concentration, a blood flow rate, a pre-replacement fluid flow rate, a dialysate or dialysis fluid flow rate, a post-replacement fluid flow rate, or a fluid removal rate.

The example plasma electrolyte management system, methods, and apparatus may determine and/or recommend changes to RRT parameters for a current RRT prescription. The example plasma electrolyte management system, methods, and apparatus may also determine or recommend RRT parameters for a new RRT prescription. The determined or recommended changes are configured to achieve a defined patient outcome, such as at least one of a target equilibrium concentration of a plasma electrolyte concentration or a pace/rate of correction of a plasma electrolyte concentration. The plasma electrolyte management system, methods, and apparatus may be configured to ensure the pace/rate of correction does not exceed a clinical threshold to avoid adverse medical conditions associated with increasing/decreasing a plasma electrolyte concentration too quickly. In some instances, the plasma electrolyte management system, methods, and apparatus may adjust or transmit instructions that cause an RRT machine to adjust a current prescription based on the determined or recommended changes to the RRT parameters.

Reference is made herein to continuous RRT. It should be appreciated that continuous RRT does not necessarily mean continuous over a 24-hour period. Instead, continuous RRT refers to long-duration RRT that may have intermittent breaks to change fluid bags or filters or permit a patient to be moved for medical testing. For example, a continuous RRT treatment may only actively provide treatment for 15 or 20 hours a day.

I. Example Plasma Electrolyte Management System

Fig. 1 illustrates a diagram of a plasma electrolyte management system 10, according to an example embodiment of the present disclosure. The system 10 includes one or more medical devices 20. In the illustrated example, an RRT machine is shown as the medical device 20. In other examples, the system 10 may include a plurality of RRT machines 20 (such as CRRT machines), a plurality of infusion pumps, a plurality of dialysis machines, a plurality of nutritional fluid delivery machines, and/or a plurality of urine collection devices.

The example RRT machine 20 includes one or more control interfaces 30 for displaying instructions and receiving control inputs from a user. The control interfaces 30 may include buttons, a control panel, and/or a touchscreen. The control interface 30 may also be configured to enable a user to navigate to a certain window or user interface on a screen of the RRT machine 20. The control interfaces 30 may also provide instructions for operating or controlling the RRT machine 20.

The example RRT machine 20 also includes a processor 40 and a memory device 50. The processor 40 is configured to control operation of the RRT machine 20. The controlled operation may include communicating with the control interface 30, controlling one or more pumps to circulate blood, pre-replacement fluid, dialysate or dialysis fluid, post-replacement fluid, and/or fluid removal. A speed of each pump may be specified by corresponding parameters in an RRT prescription, which may be stored in the memory device 50.

The memory device 50 also stores machine-readable instructions. Execution of the machine-readable instructions by the processor 40 causes the RRT machine 20 to perform the operations discussed herein. Reference to the RRT machine 20 performing certain operations herein includes the processor 40 executing the instructions to cause the operations to be performed. The memory device 50 may include random access memory ("RAM"), read only memory ("ROM"), flash memory, magnetic or optical disks, optical memory, or other storage media.

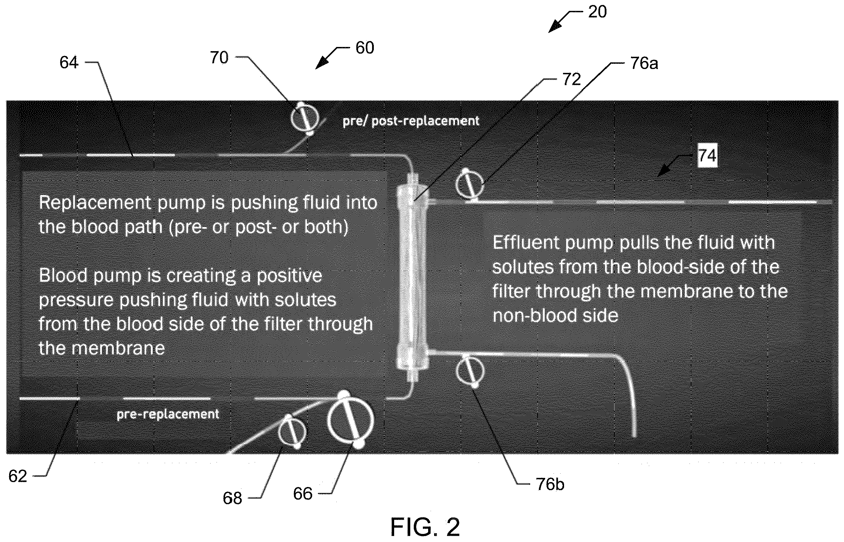

As shown in Fig. 2, the example RRT machine 20 includes an extracorporeal circuit 60 configured to circulate blood to and from a patient via arterial 62 and venous lines 64. The extracorporeal circuit 60 may also circulate pre-replacement fluid and/or post-replacement fluid to help remove toxins from the blood. The extracorporeal circuit 60 includes a first pump 66 for pumping blood at a first defined flow rate and a pre-replacement fluid pump 68 for pumping pre-replacement fluid into the blood at a second defined flow rate. The extracorporeal circuit 60 may also include (optional) a post-replacement pump 70 for pumping post-replacement fluid into the blood at a third defined flow rate. The extracorporeal circuit 60 also includes a dialyzer 72 that is configured to filter toxins (e.g., creatinine and urea) and other waste products from the blood.

The RRT machine 20 further includes a dialysate circuit 74 that is configured to circulate dialysis fluid or dialysate. The dialysate circuit 74 is connected to a fresh dialysate source and a used dialysate container or drain. In other instances, the dialysate circuit 74 may include a sorbent cartridge to cleanse the dialysate to enable its recirculation. The dialysate circuit 74 is also connected to the dialyzer 72 for removing filtered toxins and other waste products. The toxins and waste products are transferred into the dialysate, creating used dialysate or effluent. The dialysate circuit 74 includes one or more pumps 76 for moving the dialysate. A first pump 76a may pump fresh dialysate into the dialyzer 72 while a second pump 76b pulls used dialysate from the dialyzer 72.

Returning to Fig. 1, the medical device 20 is communicatively coupled to a connectivity server 102 via a network 100. The connectivity server 102 provides bidirectional communication between the medical device 20 and a system hub 104. The network 100 may include any wired or wireless network including the Internet and/or a cellular network. The network 100 may include a local area network, such as a network of a medical facility.

The example system hub 104 enables data and information concerning each medical device 20 to travel back and forth via the connectivity server 102 between the devices 20 and the other devices that are connected to the server 102. In the illustrated embodiment, the system hub 104 is connected to a clinician server 106 and an EMR database 108. In some instances, the system hub 104 and the connectivity server 102 may be implanted on a single device.

The example connectivity server 102 and enables the medical device 20 to transfer patient data 114 to and from the clinician server 106 and/or the EMR database 108 via the system hub 104. A connectivity service operating via the server 102 ensures that the connection with the device 20 is secure, ensures that the data correctly passes through the device's 20 firewalls, detects whether there has been a data or system crash, and ensures that the connectivity server 102 is communicating with the correct the medical device 20.

The example system hub 104 is communicatively coupled to the clinician server 106 and/or the EMR database 108. As described in more detail below, the clinician server 106 is configured to execute one or more instructions, routines, algorithms, applications, or programs 110 for performing the operations described herein. The EMR database 108 is configured to store RRT prescriptions 112 for patients. The prescriptions 112 for a patient may also be stored in the memory device 50 of the RRT machine 20 such that parameters of the prescription are used by the RRT machine 20 to conduct a RRT or CRRT by controlling one or more pumps and/or valves.

The EMR database 108 also stores patient data 114 in one or more medical records. As disclosed herein, the patient data 114 includes fluid input/output data that provides indications of plasma electrolyte concentrations that may be needed by the model 122. The patient data 114 may include, for example, treatment data from the medical device 20 and/or treatment data from other medical devices. The patient data 114 may also include lab results, such as test results from a blood test.

Additionally or alternatively, the patient data 114 may include demographic data that may be provided by a clinician/patient, specified within a prescribed RRT prescription, and/or provided via patient registration. The demographic data may include a patient age, a gender, a patient mobility level, a renal condition for a patient, a prescription history, etc. In some embodiments, the patient data 114 may include an identifier, which enables the EMR database 108 to store the received data in an appropriate patient record. The identifier may include a patient identifier, a patient name, and/or an identifier of the medical device 20.

As shown in Fig. 1, the clinician server 106 may be coupled to a memory device 120 that stores the one or more instructions, routines, algorithms, applications, or programs 110. The memory device 120 may also store one or more kinetic physiological models 122, discussed herein. For example, a first model 122a may be stored for plasma sodium and a second model 122b may be stores for plasma potassium. The models 122 are configured to determine a current plasma electrolyte concentration using the patient data 114 and/or the RRT prescription 112 for a given patient. The models 122 may also predict a plasma electrolyte concentration using the patient data 114 and/or the RRT prescription 112. Further, the models 122 may determine changes to RRT prescription parameters to achieve at least one of a target equilibrium concentration or a pace/rate of correction of a plasma electrolyte concentration. The memory device 120 may include random access memory ("RAM"), read only memory ("ROM"), flash memory, magnetic or optical disks, optical memory, or other storage media.

The example system 10 of Fig. 1 also includes a web portal 150 to facilitate the transmission of data to a clinician device 152 and/or a personal mobile communication device via a network 154. The example network 154 may include any wired and/or wireless network, such as the Internet and/or a cellular network. The web portal 150 may include one or more application programming interfaces ("API") that provide for the communication of the patient data 114, the RRT prescriptions 112, and/or outputs from the model 122. In some instances, the web portal 150 may be configured as a gateway device and/or firewall such that only authorized users and/or devices may communicate with the clinician server 106 and/or the EMR database 108. Further, the web portal 150 may create a separate session for each connected device 152.

The clinician device 152 may include an application 160 that is configured to interface with the web portal 150 for communicating with the clinician server 106 and/or the EMR database 108. For example, the application 160 may include one or more use interfaces with data fields that display the patient data 114, the RRT prescriptions 112, and/or outputs from the model 122. The data fields are mapped to one or more APIs at the web portal 150, which are linked to one or more data structures at the EMR database 108 and/or the clinician server 106. Selection of a user interface via the application 160, causes a request message to be transmitted from the application 160 to the web portal 150 identifying the data fields that are related to the requested user interface. The request message may also identify a patient. In response, the web portal 150 transmits one or more request messages to the clinician database 106 and/or the EMR server 108 to retrieve the patient data 114, the RRT prescriptions 112, and/or outputs from the model 122.

In other instances, the application 160 is a web browser configured to access one or more web pages via the web portal 150 that are hosted by the clinician server 106. In these other instances, the clinician server 106 provides the user interfaces and corresponding data fields in one or more web pages. A user may interact with the web browser to via desired data. The application 160 may also include native control or other installed applications on the device 152.

In some instances, the web portal 150 is configured to convert at least some of the patient data 114, the RRT prescriptions 112, and/or outputs from the model 122 from a text-based standard or Health-Level-7 ("HL7") standard (e.g., a medical standard) to a web-based message (e.g., a HTTP message, an HyperText Markup Language ("HTML") message, an Extensible Markup Language ("XML") message, a JavaScript Object Notation ("JSON") payload, etc.). In other embodiments, the connectivity server 102 is configured to convert HL7 treatment data from the medical device 20 into a text-based or web-based format (e.g., a JSON format) for processing by the clinician server 106 and storage by the EMR database 108.

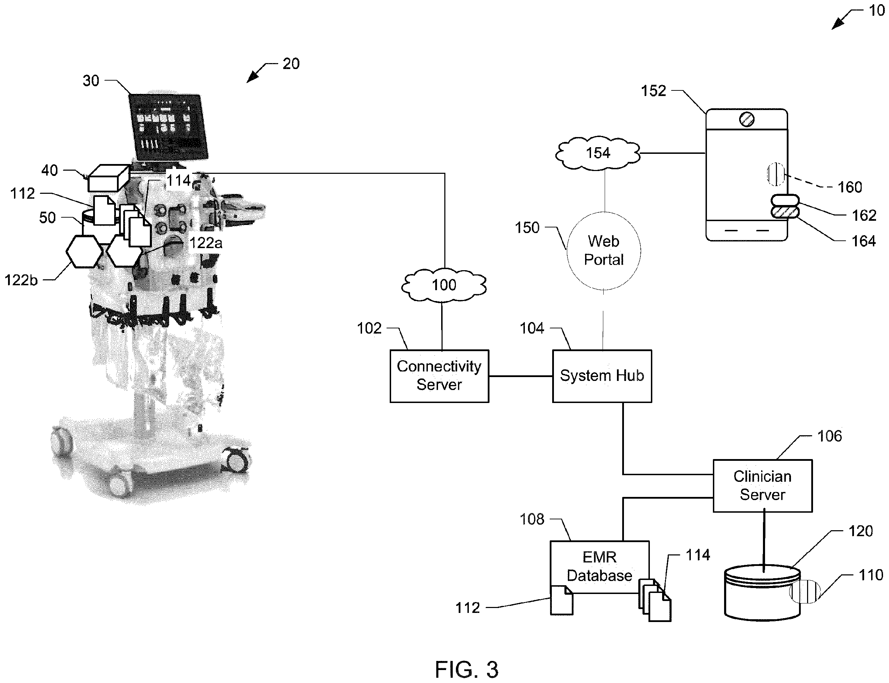

In the illustrated example of Fig. 3, the clinician device 152 includes a processor 162 that is in communication with a memory 164 storing instructions. At least some of the instructions define or specify the application 160, that, when executed by the processor 162, cause the processor 162 to provide features for displaying and interacting with the patient data 114, the RRT prescriptions 112, and/or outputs from the model 122. The processor 162 may comprise digital and analog circuity structured as a microprocessor, application specific integrated circuit ("ASIC"), controller, etc. The memory 164 includes a volatile or non-volatile storage medium. Further, the memory 164 may include any solid state or disk storage medium.

In the illustrated example of Fig. 1, the clinician server 106 is configured to execute the one or more models 122 to determine plasma electrolyte concentrations and/or change RRT prescription parameters based on the concentrations. As such, the medical device 20 (and other medical devices associated with the patient) is configured to transmit locally generated patient data 114 to the EMR database 108. The server 106 accesses one or more records associated with the patient to obtain the patient data. The server 106 then configured to transmit any changes to a RRT prescription to the medical device 20 via one or more messages using the connectivity server 102, system hub 104, and/or network 100.

In some embodiments, the clinician server 106 and/or the EMR database 108 may be configured in a distributed computing environment (e.g., a cloud computing system). The clinician server 106 may host one or more web services that apply the kinetic physiological models 122 disclosed herein to patent data 114 and/or an RRT prescription 112 for a patient. In this manner, the clinician server 106 is configured to provide data modeling as a service. The cloud-based clinician server 106 transmit outputs from the models 122 to the application 160 on the clinician device 152. Changes to the RRT prescription received from the application 160 are stored to the EMR database 108 and/or transmitted to the appropriate medical device 20.

Fig. 3 is an alternative embodiment of the plasma electrolyte management system 10, according to an example embodiment of the present disclosure. In this example, the models 122 and the patient data 114 is stored at the memory device 50 of the RRT machine 20. The models 122 may be received from the clinician server 106, which may periodically update the models 122. The patient data 114 is generated by the processor 40 during one or more RRTs and/or CRRTs. The patient data 114 for other input/output fluids and/or lab data may be accessed from the EMR database 108.

In the illustrated configuration, the RRT machine 20 is configured to use predictions about a patient's plasma electrolyte concentration to locally adjust RRT prescription parameters. In some embodiments, the RRT machine 20 may prompt an operator via the control interface 30 (or via the application 160 on the clinician device 152 via the web portal 150) to approve or accept recommended changes to the RRT prescription parameters to maintain or achieve a desired or specified plasma electrolyte concentration. In some instances, the desired plasma electrolyte is part of the RRT prescription 112. In other instances, the RRT machine 20 automatically applies the changes to a RRT prescription 112 without intervention from a clinician. The processor 40 may use one or more rate thresholds specified by the models 122 to ensure a pace/rate of correction of a plasma electrolyte concentration is within medically acceptable limits.

II. Plasma Electrolyte Concentration Modeling Embodiments

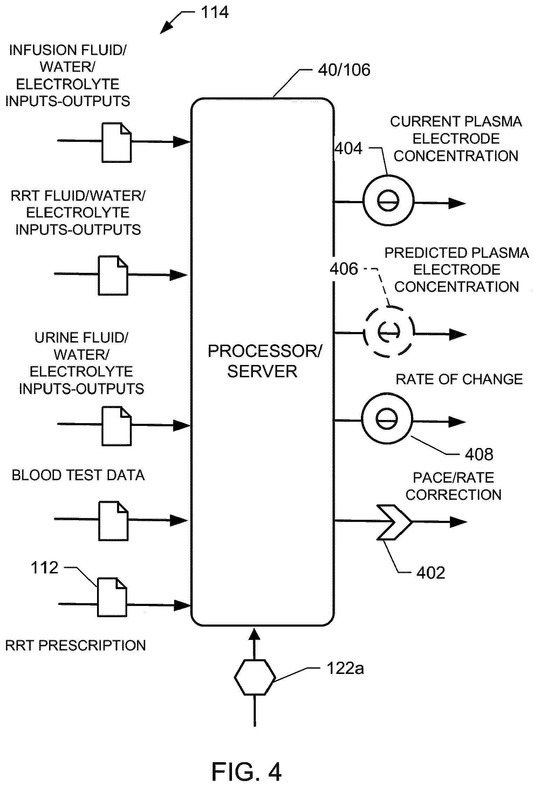

The example plasma electrolyte management system 10 of Figs. 1 and 3 is configured to model a patient's plasma electrolyte concentration using more readily data rather than requiring frequent blood testing. Fig. 4 is a diagram illustrative of the modeling performed by the plasma electrolyte management system 10, according to an example embodiment of the present disclosure. As discussed above, the example modelling is performed by the processor 40 of the medical device 20 and/or the clinician server 106 using one or more kinetic physiological models 122.

Fig. 4 shows that the processor 40 and/or clinician server 106 receives more readily available patient data 114 that is related to fluid, water, and/or electrolyte inputs/outputs. The patient data 114 may include a fluid status, a body weight, infusion data of fluids infused into a patient, dialysis data of fluids added to a patient's blood or removed from a patient, urine collection data, and/or any other data that specifies volumes of liquids and/or electrolytes removed from a patient. Generally, the content of such fluids is specified in a prescription (such as an infusion prescription) or determined by processing data related to a treatment without the need to conduct a separate blood test. The processor 40 and/or clinician server 106 also takes into account parameters of an RRT prescription 112, such as fluid rates, concentrations of electrolytes added through post-replacement fluid, and/or desired plasma electrolyte concentrations.

The example models 122 take into account estimates of the fluid and electrolytes inputs and outputs expected to occur over the prediction time window over a time period, such as four hours, eight hours, twelve hours, sixteen hours, 24 hours, 48 hours, or 96 hours, etc. This means that, even if all relevant patient data 114 can be collected automatically in real time, there is a need to anticipate about the inputs and outputs to occur. The example models 122 in conjunction with the processor 40 and/or clinician server 106 is accordingly configured to estimate future fluid and/or electrolyte inputs/outputs. As described below in regards to an electrolyte or solute rate, positive values refer to input into a patient and negative values refer to removal from a patient.

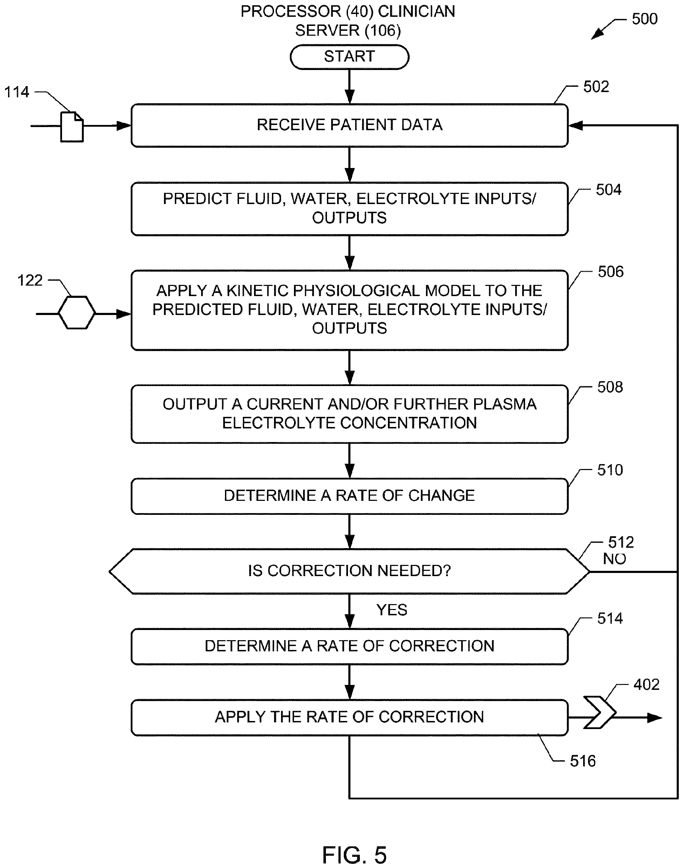

Fig. 5 is a flow diagram of an example procedure 500 to use the kinetic physiological model 122 to estimate plasma electrolyte concentrations in a patient, according to an example embodiment of the present disclosure. Although the procedure 500 is described with reference to the flow diagram illustrated in Fig. 5, it should be appreciated that many other methods of performing the steps associated with the procedure 500 may be used. For example, the order of many of the blocks may be changed, certain blocks may be combined with other blocks, and many of the blocks described may be optional. In an embodiment, the number of blocks may be changed based on uses of the model 122. Further, the steps related to determining recommendations and/or pace/rate correction parameters for an RRT prescription 112 may be omitted. The actions described in the procedure 500 are specified by one or more instructions and may be performed among multiple devices including, for example the clinician server 106 and/or the processor 40.

The example procedure 500 begins when the processor 40 and/or the clinician server 106 performs a retrospective assessment of the fluid inputs and outputs and to predict a trend over the prediction time window. This assessment includes accessing or otherwise obtaining the patient data 114 and/or the RRT prescription 112 (block 502). The assessment may also make assumptions about constant inputs and outputs according to 'recent' mean values of patient data 114 and/or predict a next evolution of each parameter on the basis of 'recent' patient data 114 to obtained a predicted evolution of non-RRT inputs/outputs over the time window (e.g., non-constant value over time, trending analysis) (block 504). A reasonable alternative to the detailed analysis of all fluid inputs and outputs is to use the prescribed patient net fluid balance target over the prediction time window as the patient data 114, in some embodiments. The patient net fluid balance is a meaningful clinical target that is managed for acute and other intensive care patients on RRT, as controlled via the patient fluid removal rate prescribed through an RRT prescription 112. The patient net fluid balance matters with respect to changes of the patient distribution volume over time.

Similarly to fluid/water, electrolyte inputs and outputs can be estimated over the prediction time window from an analysis of the recent retrospective data. This requires collection of the electrolyte concentration within each of the fluid inputs/outputs (e.g. sodium content of infusion fluids) including electrolyte concentration in urine output (or other fluid losses) as further patient data 114. In this context, the data collection process may include assumptions by default that non-RRT electrolytes inputs/outputs are balanced towards a physiologic electrolyte content (e.g., 140 mM for sodium). The data collection process may also include querying the patient data 114 for the presence of significant fluid input or output having an electrolyte content far from the physiologic value (e.g., glucose infusion with no sodium).

The example processor 40 or clinician server 106 is configured to apply one or more kinetic physiological models 122 to the predicted fluid, water, and electrolyte inputs/outputs for the given time window to estimate current and future plasma electrolyte concentrations (block 506). While 2-compartment models (intra and extracellular spaces) are typically required for accurate simulation of sodium changes during intermittent hemodialysis ("IHD"), a simpler single-compartment model may be acceptable in the context of CRRT applications where slow sodium concentration changes are needed for safe correction of major dysnatremias. With this single-compartment approach, the apparent sodium distribution volume can be estimated through the model 122 using total body water ($V_{tot}$) and/or total body water corrected according to sodium and potassium levels. It should be noted that the dependence of apparent sodium distribution volume on potassium means that some control of potassium may be required for accurate control of severe dysnatremia cases. Simultaneous correction of sodium and control of potassium levels are possible in a model 122 where both sodium and potassium content of the fluid(s) can be adjusted on demand (and varied over time).

In the context of intensive care, patients are frequently fluid overloaded, especially when dealing with patients prescribed for RRT. Dehydration may also be faced by some of these patients. In these situations, the distribution volume may be corrected for this deviation from normo-hydration status (ΔVhydr). While this correction is straightforward in the single-compartment approach, the 2-compartment approach requires further assumptions on how the fluid volume deviation splits across the intra and extra-cellular compartments. Another aspect of ICU patients receiving CRRT is significant hemodilution increasing the water volume of the intravascular compartment. In the context of the examples provided herein, the single-compartment approach is taken using the total body water as apparent sodium distribution volume and ignoring possible biases from potassium, as provided in equation (1) below:

$$Vdis = V_{tot} + \Delta Vhydr = V_{intracellular} + V_{extracellular} + \Delta Vhydr$$

$$Vdis = 0.60 \times BWref + \Delta Vhydr \tag{1}$$

Definitions for the above variables and further variables discussed are provided below:

$BW_{ref}$ reference patient body weight;
ΔVhydr patient fluid volume deviation from reference body weight (signed);
$V_{corr}$ expected change in ΔVhydr over a prediction period;
Vdis electrolyte distribution volume;
$V_{extracellular}$ extracellular electrolyte distribution volume;
$V_{interstitial}$ interstitial electrolyte distribution volume;
$V_{vascular}$ vascular electrolyte distribution volume;
$V_{fluid\_in}$ volume of infusion fluids;
$V_{fluid\_out}$ volume of fluid losses;

$V_{PFR}$ fluid removal volume from the RRT system;

Cp patient plasma electrolyte concentration;

$Cp_{inlet}$ electrolyte plasma concentration at a hemodialyzer/filter inlet;

$Cp_{memb}$ effective electrolyte concentration for membrane mass transfer;

Cpw patient plasma water concentration;

Cd dialysate concentration;

Cpre pre-replacement fluid concentration;

Cpost post-replacement fluid concentration;

Fp plasma water fraction;

Hct hematocrit;

$J_{RRT}$ net solute balance rate from an RRT therapy (signed);

$J_{non-RRT}$ net solute balance rate from all electrolyte inputs and outputs other than originated from the RRT therapy (signed);

K electrolyte hemodialyzer/filter clearance;

K0 electrolyte mass transfer coefficient;

A hemodialyzer/filter surface area;

Qb blood flow rate;

Qp plasma flow rate;

Qpw plasma water flow rate;

Qd dialysate flow rate;

Qpre pre-replacement flow rate;

Qpost pre-replacement flow rate;

Qeff effluent flow rate;

QPFR patient fluid removal rate (on the RRT system);

Qnet correction rate of the patient fluid volume (signed); and

Tpred duration of the prediction time window.

Correcting the fluid balance status of an acute or ICU patient under RRT is a critical and complex process having to address periodic assessment of fluid inputs/outputs and decide on the RRT fluid removal rate prescription ($Q_{PFR}$) as to bring the patient to a normal hydration status over time. While the fluid overload frequently encountered in ICU patients initiated on CRRT is critical to outcome and needs to be corrected, excessive fluid removal is associated with a high risk of hypotensive episodes. In the context of a streamlined data collection process where the expected/prescribed patient volume correction ($V_{corr}$) over the prediction time period is collected instead of all the fluid inputs/outputs details, the model 122 disclosed herein is configured to derive the difference between the fluid inputs and outputs and an estimate of the non-RRT sodium (electrolyte) infusion/losses. This is expressed through following equations (2) to (5) specifically applicable to the prediction time period (Tpred).

$$V_{corr} = V_{fluid\_in} - V_{fluid\_out} - V_{PFR} \quad (2)$$

$$V_{fluid\_in-out} = V_{fluid\_in} - V_{fluid_{out}} = V_{PFR} + V_{corr} \quad (3)$$

$$J_{non-RRT} \approx \frac{V_{fluid_{in}-out} \times C_{default}}{Tpred} = (Q_{PFR} + Q_{net}) \times C_{default} \quad (4)$$

$$Q_{net} = \frac{V_{corr}}{Tpred} \quad (5)$$

It should be noted that Vcorr and Qnet are signed parameters, with a negative value in case of fluid removal from the patient or a positive value in the less common case of net fluid gain. The $Q_{net}$ variable is an 'internal' parameter to the model that is used to calculate a pace/rate correction value 402 over the prediction time period (Tpred). It should also be noted that $C_{default}$ is an assumption made on the typical concentration of non-RRT fluid inputs/outputs (e.g., a physiologic concentration), possibly adjusted according to the presence of significant non-physiologic electrolyte inputs or outputs.

Kinetic changes of the patient sodium plasma concentration can be described through the following set of equations of the model 122.

$$
\left\{
\begin{array}{l}
\dfrac{d(Vdis)}{dt} = Q_{fluid\_in} - Q_{fluid_{out}} - Q_{PFR} = Q_{net} \\[2mm]
\dfrac{d(Vdis \times Cp)}{dt} = J_{RRT} + J_{non-RRT} \\[3mm]
\text{Initial conditions at time t=0:} \\
Cp = Cp0 \\
\Delta Vhydr = \Delta Vhydr0
\end{array}
\right.
\tag{6}
$$

With $J_{RRT} = A * Cp + B$

Where $A$ and $B$ are constant function of operating flow rates. When assuming $Q_{net}$, A, B and $J_{non-RRT}$ as constant over the prediction time window, the above equations can be integrated into an analytical solution for the patient plasma electrolyte concentration $Cp$ (shown in Fig. 4 as the current plasma electrolyte concentration 404 for a prediction time period (Tpred) of 0 and a predicted plasma electrolyte concentration 406 for a prediction time period (Tpred) that is greater than 0):

$$
Vdis = 0.60 \times BWref + \Delta Vhydr0 + Q_{net} \times t = Vdis0 + Q_{net} \times t
\tag{7}
$$

$$
\text{If } Q_{net} <> 0 : \qquad Cp(t) = (Cp0 + D) \times \left(1 + \frac{Q_{net}}{Vdis0} \times t\right)^{\left(\frac{A}{Q_{net}} - 1\right)} - D
$$

$$
\text{If } Q_{net} = 0 : \qquad Cp(t) = (Cp0 + D) \times e^{\left(\frac{A \times t}{Vdis0}\right)} - D
$$

$$
\text{With} \quad D = \frac{B + J_{non-RRT}}{A - Q_{net}}
\tag{8}
$$

In the case, any of the said terms is not constant, the equations can be integrated numerically to provide for the predicted evolution of patient sodium (or other electrolyte) concentration over time. A electrolyte rate of change value 408 may be determined by comparing the current plasma electrolyte concentration 404 to the predicted plasma electrolyte concentration 406 and/or a past plasma electrolyte concentration 406 to calculate how the plasma electrolyte concentration has changed (or will change) over time.

Returning to Fig. 5, after using the model 122, the example processor 40 and/or the clinician server 106 is configured to output the current and/or projected plasma electrolyte concentration (block 508). This may include displaying the plasma electrolyte concentration in the control interface 30 of the RRT machine 20 and/or via the application 160 on the clinician device 152. The example processor 40 and/or the clinician server 106 may use the current and/or projected plasma electrolyte concentrations (and/or previous plasma electrolyte concentrations) to determine an electrolyte rate of change for the plasma electrolyte concentration (block 510). The electrolyte rate of change indicates, for example, whether a sodium concentration may be decreasing contrary to a desired clinical target.

The processor 40 and/or the clinician server 106 use the electrolyte rate of change and/or the current and/or projected plasma electrolyte concentrations to determine if a correction is needed (block 512). In this step, the current plasma electrolyte rate of change may be compared to a clinically acceptable threshold for how rapidly the electrolyte may change without risks to the patient. Alternatively, the current or projected plasma electrolyte concentrations may be compared to a clinical target. It should be appreciated that this step may only be performed if a clinical target for a plasma electrolyte concentration is received or if a clinical threshold for how rapidly the plasma electrolyte may change without risk has been received. Otherwise if a clinical target is not received, the procedure 500 may end. If the values are within prescribed targets or clinical guidelines, the example procedure 500 returns to block 502 when additional patient data 114 is received or otherwise generated. However, if a correction is needed, the processor 40 and/or the clinician server 106 determine new or adjusted RRT prescription parameters needed to achieve a target the electrolyte concentration (block 514). The processor 40 and/or the clinician server 106 may adjust the RRT prescription parameters as needed to ensure a predicted future pace/electrolyte rate of change 402 does not exceed clinical thresholds. Alternatively, the processor 40 and/or the clinician server 106 provide information indicative of possible changes to the RRT prescription parameters, and enable the clinician to make such changes by, for example, enabling the clinician to validate the RRT prescription parameters before programing the RRT machine 20.

In other embodiments, the processor 40 and/or the clinician server 106 receives adjusted RRT prescription parameters from a clinician via the application 160 and/or the control interface 30. The processor 40 and/or the clinician server 106 then apply the RRT prescription parameters (block 516). This may include updating the RRT prescription parameters 112, such as a blood flow rate, a pre-replacement flow rate, a pre-replacement electrolyte concentration, a post-replacement electrolyte concentration, a dialysate electrolyte concentration, a dialysate flow rate, a post-replacement flow rate, and/or a fluid removal rate. The processor 40 and/or the clinician server 106 may automatically update the RRT prescription parameters or prompt a clinician to confirm the update. The example procedure returns to block 502 when new patient data 112 is generated or received.

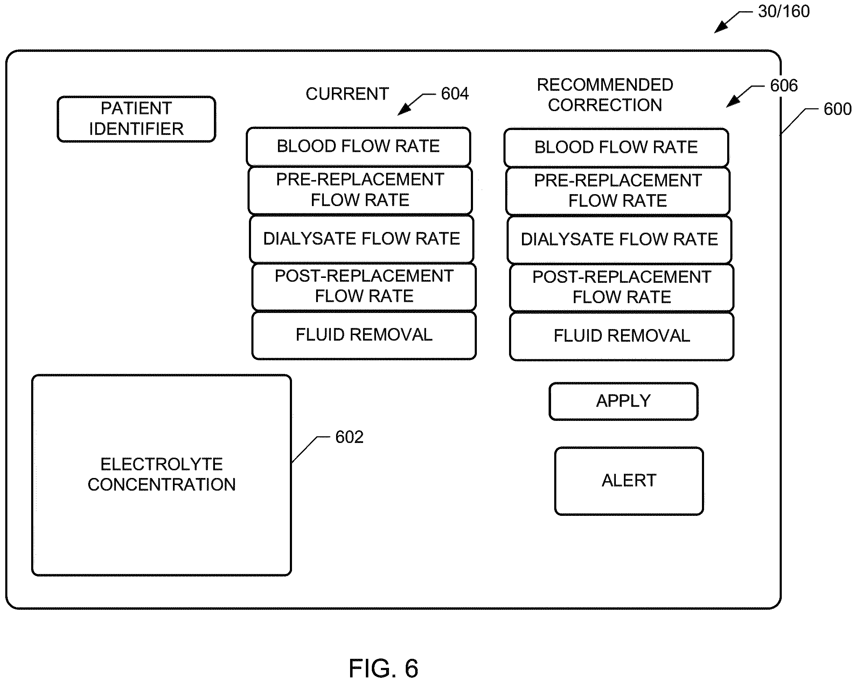

Fig. 6 is an example user interface 600 that displays plasma electrolyte concentration information for a clinician, according to an example embodiment of the present disclosure. The user interface 600 may be displayed by the application 160 on the clinician device 152 and/or at the control interface 30 of the medical device 20. The example user interface 600 includes a patient identifier. The user interface 600 may also display at least some patient data 114 (not shown). Additionally, the user interface 600 provides a display area 602 to show current and/or predicted plasma electrolyte concentrations. The display area 602 may also show a last (or previous) electrolyte measurement or prompt a clinican to manually enter an electrolyte measurement value. The user interface 600 may also show how a recommended adjustment changes the predicted plasma electrolyte concentration over a predicted time period.

The user interface 600 may also display current RRT prescription parameters 604 and recommended RRT prescription parameters 606. The recommended parameters 606 may be used to apply a rate/pace of correction to the plasma electrolyte concentration. The parameters 604 and/or 606 may also include a fluid electrolyte concentration (or fluid identifiers) and/or as fluid flow rates. In some embodiments, the parameters 606 may be time based such that first parameters are applied until a certain time duration elapses and/or the plasma electrolyte concentration reaches a certain level, at which point second, different RRT prescription parameters are applied. A clinician may use the interface 600 to approve the recommended parameters, change one or more parameters, or decline a change to a current RRT prescription. In some embodiments, the user interface 600 may also display an alarm/alert when a plasma electrolyte concentration level is above or below a threshold and/or if an electrolyte rate of change exceeds a threshold. Additionally or alternatively, the user interface 600 may include a section that compares a predicted versus a measured electrolyte concentration. The user interface 600 may display an alarm/alert if the measured electrolyte concentration values deviate above a threshold from the the measured values.

## A. Example Sodium Model

The following section develops at least some components of the model 122, which estimates the electrolyte balance $J_{RRT}$ (net gain or net removal) of an RRT procedure. Schematically, the RTT procedure can be assessed as the combination of (a) multiple electrolyte/solute infusion steps including pre-replacement (fluid infused upstream the dialyzer), dialysate, post-replacement (fluid infused downstream the dialyzer), and (b) solute removal in the effluent fluid leaving the hemodialyzer/filter. The clinician server 106 and/or the processor 40 is configured to compute the solute removal in effluent requires by computing the electrolyte concentration at the hemodialyzer/filter inlet, further to the (optional) pre-dilution infusion step(s) and use the model 122 for the hemodialyzer/filter clearance.

Solute concentration at the filter/dialyzer inlet results from the dilution of blood with pre-replacement fluid. Depending of the solute/electrolyte, the solute dilutes in the whole blood water (plasma and intraerythrocyte spaces) or only in the plasma water. According to the low concentration of sodium in the red blood cells, a good approximation for sodium is to consider that the dilution effect is restricted to the plasma compartment. This leads to the below expression of plasma sodium inlet concentration.

$$Qp \times Cp + Qpre \times Cpre = (Qp + Qpre) \times Cp_{inlet}$$

$$Cp_{inlet} = \frac{(1 - Hct) \times Qb \times Cp + Qpre \times Cpre}{(1 - Hct) \times Qb + Qpre} \qquad (9)$$

With respect to the transfer of charged solutes through the membrane, the effective concentration is derived from the plasma solute concentration considering: that proteins account for a significant fraction of the plasma volume and that the solute is actually distributed in the plasma water, and that proteins also impact the transfer of charged solutes as a consequence of their own electrical charge. This effect is integrated through the Donnan coefficient ($\alpha$). These two effects are integrated in the following equation defining the effective electrolyte concentration for membrane mass transfer:

$$Cp_{memb} = \alpha \times Cpw = \frac{\alpha}{Fp} \times Cp \qquad (10)$$

It should be noted that both plasma free fraction and the Donnan coefficient are dependent on the concentration of total proteins and albumin, and some dependence equations may be derived from literature. At the opposite side of such a detailed analysis, it can be observed that $Fp$ and $\alpha$ have similar values and that their ratio remains close to 1.0 in most circumstances. A simpler/streamline version of the model 122 would thus consider plasma electrolyte concentration $Cp$ as the effective concentration to mass transfer.

For a wide range of CRRT prescriptions, full equilibrium between blood inlet and effluent is reached, matching with a filter/dialyzer clearance equals to the effluent flow rate.

$$K = Qeff \qquad (11)$$

As a consequence of above considerations and equations, the solute balance from the extracorporeal RRT circuit can be expressed with following equations (in the context of a simplified model 122):

$$J_{RRT} = J_{RRT\_inf} - J_{eff}$$
$$J_{RRT\_inf} = Qpre \times Cpre + Qd \times Cd + Qpost \times Cpost$$
$$J_{eff} = Qeff \times Ceff = Qeff \times Cp_{memb_{inlet}} \approx Qeff \times Cp_{inlet} \qquad (12)$$

With respect to the generic expression $J_{RRT} = A{*}Cp + B$ discussed above, constants $A$ and $B$ are thus defined as:

$$A = -Qeff \times \frac{(1 - Hct) \times Qb}{(1 - Hct) \times Qb + Qpre}$$
$$B = J_{RRT\_inf} - Qeff \times \frac{Qpre}{(1 - Hct) \times Qb + Qpre} \times Cpre$$
$$B = \left( Qpre - Qeff \times \frac{Qpre}{(1 - Hct) \times Qb + Qpre} \right) \times Cpre + Qd \times Cd + Qpost \times Cpost \qquad (13)$$

B. Another Example Sodium Model

The general model for electrolyte balance in the RRT circuit is identical to the simplified model 122 discussed above except for the expression of the filter/dialyzer clearance, which does not assume full equilibrium between effluent and blood inlet. The general expression for electrolyte clearance is derived from literature, which is fully applicable to electrolytes which small size warrants no measurable sieving (sieving coefficient =1). Other assumptions related to the below equations include that (a) sodium is mainly present in plasma and no significant transfer between plasma and red blood cells is assumed while blood flows through the hemodialyzer/filter, (b) Donnan coefficient is taken as a constant along the filter (impact of increasing concentration of proteins is neglected), and (C) an evolution of the plasma water fraction along the filter/dialyzer is taken into account by referring to the plasma water flow (instead of the whole plasma flow).

$$\gamma = exp\left(\frac{Qfil}{K0 \times A}\right) - 1$$

$$f = \left(\frac{Qpw_{inlet} - Qfil}{Qpw_{inlet}} \times \frac{Qdial + Qfil}{Qdial}\right)^{1/\gamma}$$

$$K = \frac{Qpw_{inlet} \times Qdial - f \times (Qpw_{inlet} - Qfil) \times (Qdial + fil)}{Qdial - f \times (Qpw_{inlet} - Qfil)}$$

$$Qfil = Qpbp + Qpre + Qpost + Qpfr$$
$$Qpw_{inlet} = Qpw + Qpre = Fp \times (1 - Hct) \times Qb + Qpre \qquad (14)$$

The mass transfer coefficient K0 for sodium may be considered as identical to the Urea mass transfer coefficient in case no specific data is available. Previous expression of the electrolyte removal rate in the effluent may be revised in this more general context where full equilibrium with blood inlet is not taken for granted, as provided below:

$$Jeff = Qeff \times Ceff = Qd \times Cd + K \times (\alpha \times Cpw_{inlet} - Cd) + Qfil \times Cd$$

$$\text{With} \quad Cpw_{inlet} = \frac{Qpw \times Cpw + Qpre \times Cpre}{Qpw + Qpre} \qquad (15)$$

The other equations discussed above expressing $J_{RRT}$ and $J_{RRT\_inf}$ remains unchanged.

With respect to the generic expression $J_{RRT} = A*Cp + B$ discussed above, constants *A* and *B* are thus defined as:

$$A = -K \times \frac{\alpha}{Fp} \times \frac{Qpw}{Qpw + Qpre}$$

$$B = J_{RRT\_inf} - Qd \times Cd - K \times \left(\alpha \times \frac{Qpre \times Cpre}{Qpw + Qpre} - Cd\right) - Qfil \times Cd$$

$$B = \left(Qpre - K \times \alpha \times \frac{Qpre}{Qpw + Qpre}\right) \times Cpre + (K - Qfil) \times Cd + Qpost \times Cpost$$

$$(16)$$

III. Example Plasma Electrolyte Modeling Applications

The following sections describe some plasma electrolyte modeling applications. It should be appreciated that the example applications are non-limiting and only provide context regarding the capabilities of system, methods, and apparatus disclosed herein.

A. Patient Trajectory Application Embodiment

In one application, the system, methods, and apparatus described herein offers the prescribing clinician with a predicted evolution of patient electrolyte concentration over time. More specifically the system, methods, and apparatus can provide for various parameters of specific interest such as: Time to reach a predefined electrolyte concentration, Concentration level(s) reached at predefined time(s), Rate of patient electrolyte concentration change (mmol/L/h) at predefined time(s), and/or maximal predicted change rate. To get this predicted patient evolution, the model parameters described in previous sections are needed. Depending on the implementation configuration the example system, methods, and apparatus may automatically gather the RRT parameters as well as some other parameters as the patient data 114, and determine a predicted patient trajectory computed from the time of the last patient concentration measurement. Alternatively, or in addition, the system, methods, and apparatus can offer the prescribing clinician for comparative data between two different prescriptions, one of them being optionally the current prescription.

This first example illustrates the application where the system, methods, and apparatus predict only for the evolution of patient electrolyte concentration over time as a function of the set RRT parameters. As therapy guidelines provide for some empirical limits to the safe rate of correction of sodium concentration (e.g. not more than 8 mmol/L per day), the system, methods, and apparatus also illustrates for this correction rate. It should be noted that the threshold rate value may be user settable. In addition, this criterion may not be of relevance for other electrolytes for which other empirical safety criteria may apply and be implemented in the system, methods, and apparatus.

Table 1 : parameters for kinetic sodium modelling - example A1

| Patient data | | | RRT data | | |
|---|---|---|---|---|---|
| BWref (kg) | 78 | | parameter | Flow rate* | [Na] (mM) |
| Hct (%) | 31 | | blood | 180 | N/A |
| Sodium kinetic data | | | Pre-replacement | 500 | 140 |
| Cp0 (mM) | 157 | | Dialysate | 1000 | 140 |
| ΔVhydr0 (L) | 4.6 | | Post-replacement | 800 | 140 |
| Prediction time (h) | 24 | | Fluid removal | 120 | N/A |
| Vcorr (L) | -1.8 | | | | |
| *ml/min for blood, ml/h for other flow rates* | | | | | |

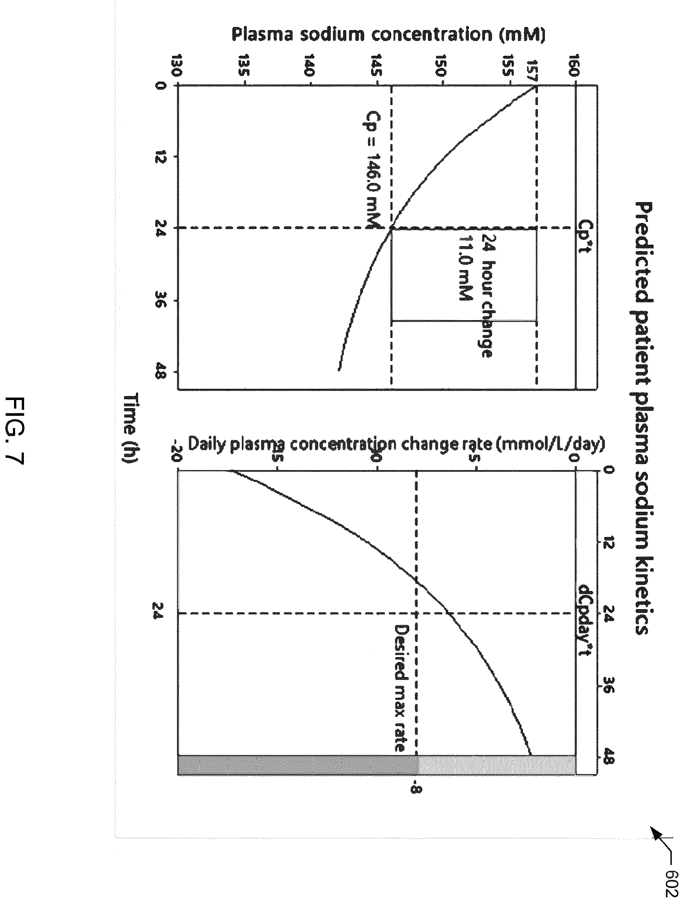

Table 1 shows patient data 114 that may be readily available without the need for multiple blood tests. Table 1 also shows RRT prescription parameters 112 for a current prescription. Fig. 7 shows a computed patient plasma sodium kinetics over twice a prediction time. The left panel shows a patient sodium profile with predicted value at the prediction time and the concentration shift over the period. The right panel shows an evolution of the daily patient plasma concentration change rate over time, with the optional identification of a threshold. According to the set RRT parameters, the system, methods, and apparatus predict more than half sodium correction over the first day. It also highlights the correction pace is significantly higher than the desired value. This observation indicates that the RRT prescription may not be adequate.

B. Comparison of two RRT Prescriptions Application Embodiment

Further to the results from the first application, it becomes relevant to evaluate the impact of modified RRT parameters as to get a slower correction of the patient hypernatremia state. The system, methods, and apparatus may thus offer for the option to compare two (or more) prescriptions. In this example, results from the first example are compared to an alternative RRT prescription where the prescribing clinician considers reducing significantly the CRRT dose as to slow down the sodium correction.

Table 2 : RRT parameters for the alternative prescription to example A1 - changes in colored cells

| RRT data - example A2 | | |
|---|---|---|
| parameter | Flow rate* | [Na] (mM) |
| blood | 180 | N/A |
| Pre-replacement | 200 | 140 |
| Dialysate | 500 | 140 |
| Post-replacement | 300 | 140 |
| Fluid removal | 120 | N/A |
| *ml/min for blood, ml/h for other flow rates* | | |

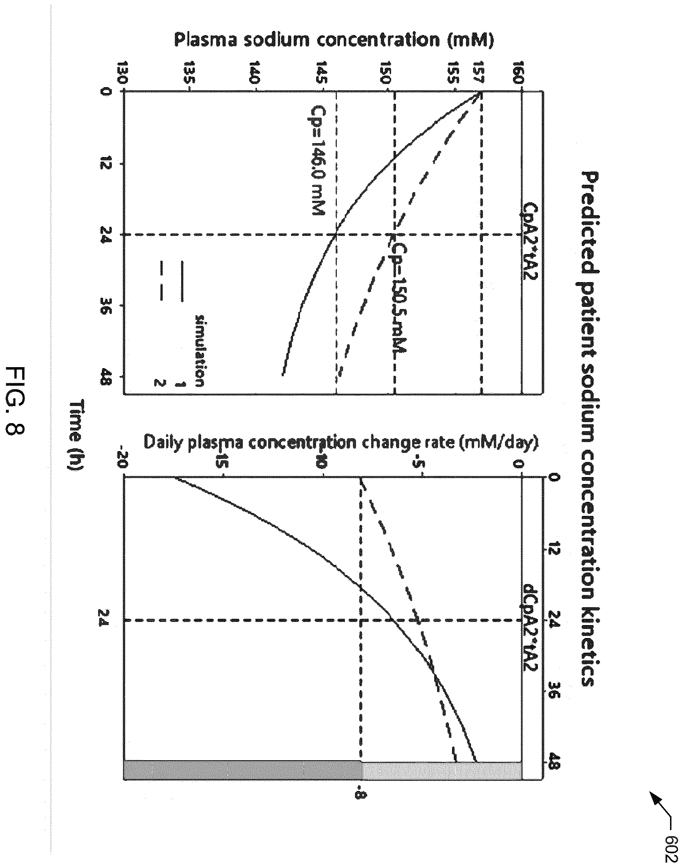

Figure 8 shows a comparison of two RRT prescriptions for patient sodium kinetics for this example. Results from the application indicate that a sodium correction rate of the new prescription with minimal RRT dosing (about 15 ml/kg/h) meets requirements for safe sodium correction at any time.

C. On-going CRRT Therapy and Correction for Sample Time Application Embodiment

This example illustrates the capacity of the systems, methods, and apparatus to take into account the collection/measurement time of some parameters in a scenario of on-going RRT treatment. This example is built for the same patient of the previous two examples and assumes on-going CRRT treatment according to the RRT conditions of the first example (see Table 1). Requested simulation of new CRRT conditions is based on the RRT parameters of the second example.

Table 3 : time data associated to the examples A1 to A3

| Parameter | Time definition | Example A1-A2 | Example A3 |
|---|---|---|---|
| Cp0 (mM) | Blood sample time | 9:00 (day 1) | 6:30 (day 1) |
| Vcorr (L) | Time window associated to the fluid volume correction | 9:00 (day 1) to 9:00 (day 2) | |
| Time origin for prediction | | 9:00 (day 1) | |

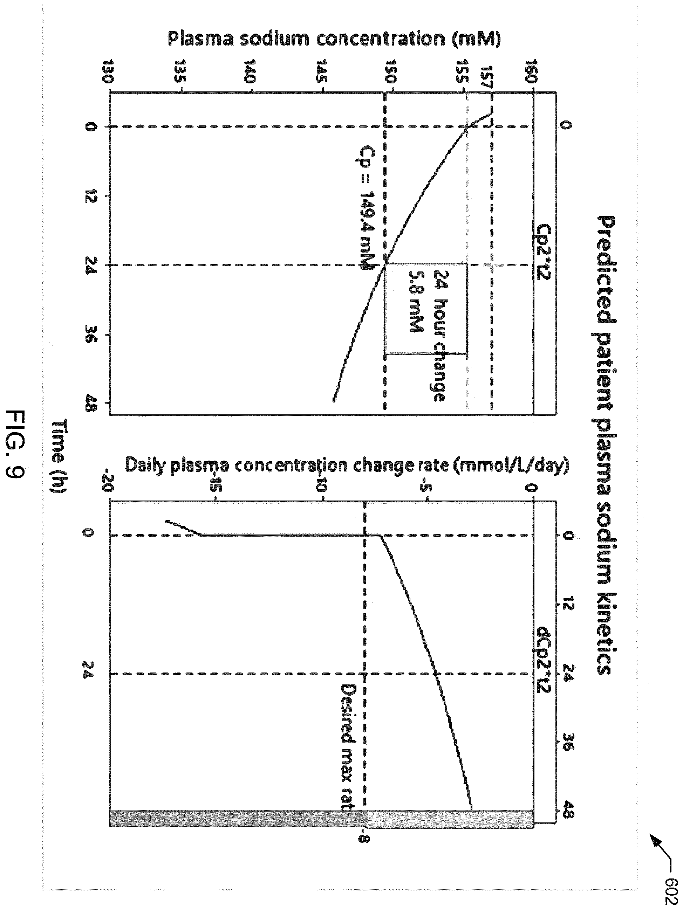

Figure 9 shows consideration of a bias from on-going RRT treatment according to the time delay of patient blood sampling and simulation start. This example illustrates the significant bias which can occur from on-going RRT treatment (and patient electrolyte concentration changes) on the outcome of the simulation. In the second example, the RRT conditions leads to a 6.5 mM decrease of patient concentration over the first 24 hours from the set starting point of 157 mM. In this third example, the same RRT conditions leads to a 5.8 mM decrease when considering the effect on the on-going RTT procedure on the patient sodium concentration (estimated as 155.3 mM when moving to the CRRT parameters of the second example).

D. RRT Parameters to Achieve a Prescribed Target Application Embodiment

In this example, the same kinetic model 122 as described above can be used by an algorithm executed by the processor 40 or the clinician server 106 to define RRT prescriptions that would deliver against a predefined clinical target set by the prescribing clinician. This clinical target might be expressed as: The target patient electrolyte concentration to be reached ($C_{target}$), and the time for reaching the target concentration and/or the maximum change rate of patient concentration.

The system, methods, and apparatus may accept different adjustable RRT parameters, upon a prescribing clinician's choice including RRT dose (e.g. Urea clearance) and flow rates and/or electrolyte concentration in fluid(s). The system, methods, and apparatus may offer as well for the definition of acceptable boundaries to the adjustable parameters. Depending on the current patient state, set target and adjustable parameters, the algorithm may provide for different outputs including one single prescription in the case of one single adjustable parameter (e.g. dialysate sodium concentration), several prescription options which could be ranked according to predefined criteria, and/or an indication that it is not possible to deliver for the target within the allowed adjustable ranges of parameters.

In this example, one or more RRT parameter is/are computed according to a clinical target.

Table 4 : B1 example with adjustable dialysate sodium concentration in CVVHD

| Patient data | | | RRT data | | |
|---|---|---|---|---|---|
| BWref (kg) | 91 | | parameter | Flow rate* | [Na](mM) |
| Hct (%) | 26 | | blood | 220 | N/A |
| Sodium kinetic data | | | Pre-replacement | 0 | - |
| Cp0 (mM) | 118 | | Dialysate | 2500 | Adjustable |
| ΔVhydr0 (L) | 7.2 | | Post-replacement | 0 | - |
| Vcorr (L) | -0.5 | | Fluid removal | 150 | N/A |
| Prediction time (h) | 24 | | | | |
| Cp$_{target}$ (mM) | 126 | | | | |
| * ml/min for blood, ml/h for other flow rates | | | | | |

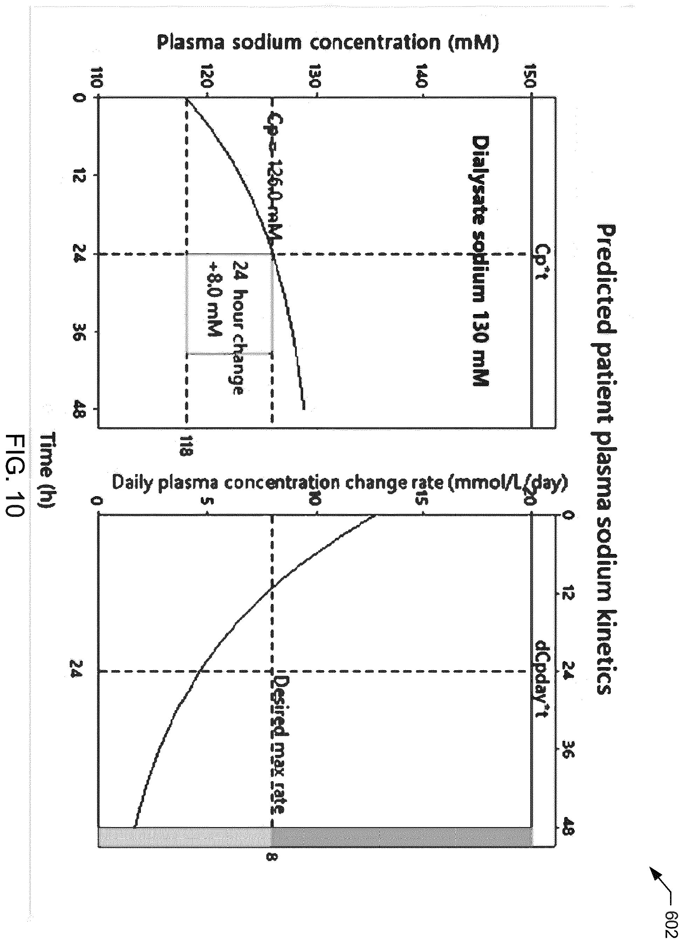

Figure 10 shows a model output for this example with a definition of the dialysate sodium concentration allowing to reach the set patient plasma sodium level at the end of the prediction time window. As to achieve the sodium target defined in Table 4, a dialysate sodium concentration of 130 mM is required. Although the overall expected sodium patient shift is 8.0 mM over the first 24 hours, the model 122 shows that the initial change pace is significantly higher, in the range of + 13 mM/day during the first treatment hour.

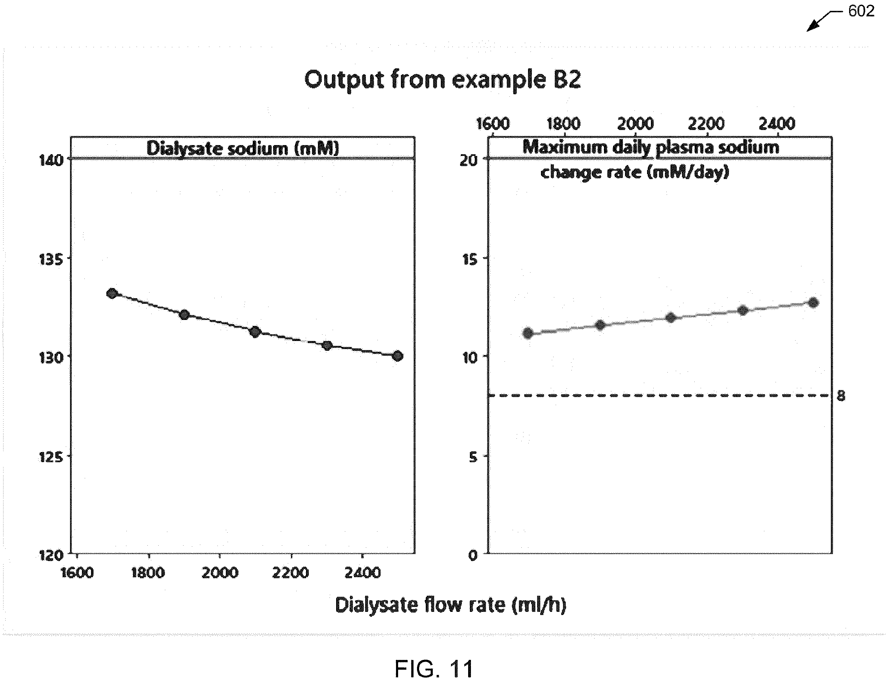

In another example, same parameters as the above example may be maintained with both adjustable dialysate flow rate and sodium concentration. A minimum dialysate flow rate value of 1700 ml/h is defined as to secure a minimum RRT dose. Fig. 11 shows a model output for this example showing the combination of dialysate flow rate and dialysate sodium concentration delivering on the set clinical target (Cp=126 mM at 24 h). This additional example shows that the required dialysate sodium concentration is minimally dependent on the dialysate flow rate in the specific context. The 3.2 mM computed difference in sodium dialysate concentration between min and max flow rate 1700 and 2500 ml/h matches with about 2% variation of the sodium concentration. While these recommended dialysate sodium concentration adjustments may be ignored in practice, the model 122 however show more relevant dependence of the plasma sodium change rate on the dialysate flow, which may be considered for the clinical application.

### E. RRT Parameters with an Upper Change Rate Limit Application Embodiment

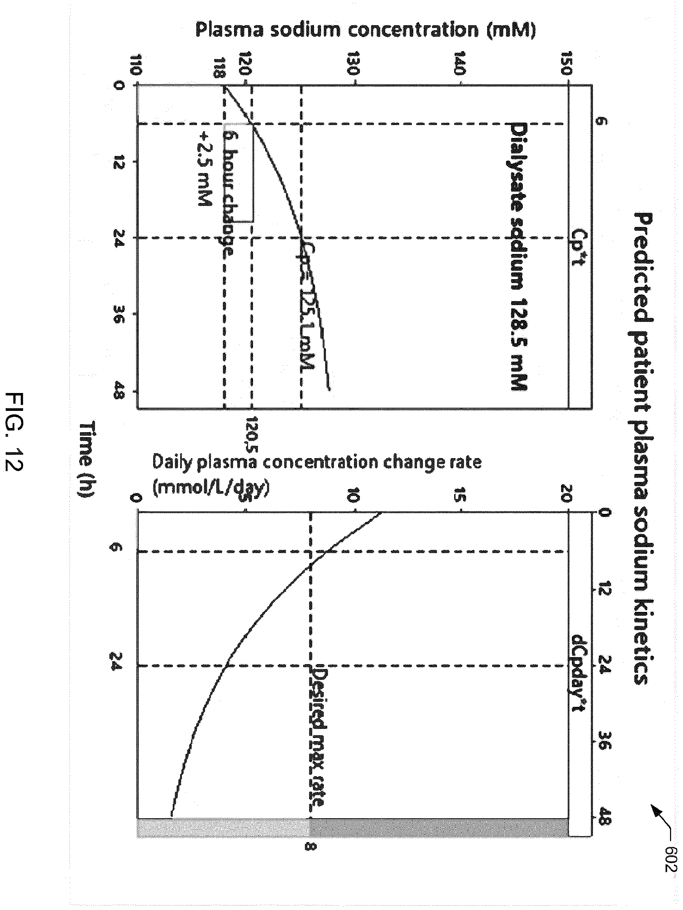

This example builds off the previous example except for the expression of the clinical target. In this example, the target concentration at time $T_{pred}$ is replaced by the definition of a maximum plasma concentration change rate, which is expressed as: maximum Cp change of 2.5 mM over the 6 first hours of RRT. When extrapolated to 24 hours, the set rate would match with 2.5/6x24 = 10 mM/day. Figure 12 shows a model output for this example with a definition of the dialysate sodium concentration enables the system to keep the patient sodium drift below 2.5 mM after 6 hours. As expected from the previous simulation results discussed above, a lower dialysate concentration of ~128 mM is needed to keep the patient sodium shift within the specified limits at the start of the RRT treatment.

### F. Integration of Monitoring Patient Data Over Time Application Embodiment

In some embodiments, the example model 122 is configured to take advantage of several patient electrolyte measurements as to: a check that patient kinetics match with a predicted trajectory, and generate an alert on inconsistencies in case of excessive deviations between the predicted and observed data. The electrolyte measurement may originate from the periodic collection of blood samples (e.g. periodic blood gases analysis providing as well for electrolytes) or from an in-line measurement device integrated to the RRT system.

In this example, the RRT prescription parameters may be similar to the examples discussed above. It is assumed that the RRT therapy has been started at time zero with a computed dialysate concentration of 130 mM. After 6 hours, a measurement of patient plasma sodium is made available. The next table shows typical outputs/actions from the system disclosed herein according to the measured value. In the table below, the data is indicative of a management of monitoring data collected during the prediction time. The patient data 114 shows expected patient sodium at 6 h according to variations within expected data accuracy.

| Dialysate sodium (mM) | Initial patient plasma sodium (mM) | | | | |
|---|---|---|---|---|---|
| | 116 | 117 | 118 | 119 | 120 |
| 129 | 119.1 | 119.8 | 120.6 | 121.4 | 122.2 |
| 130 | 119.3 | 120.0 | 120.8 | 121.6 | 122.4 |
| 131 | 119.5 | 120.3 | 121.0 | 121.8 | 122.6 |

The table below shows system response according to the measured value at 6 hours (assuming no decimal available).

| Case | Measured value (mM) | System response |
|---|---|---|
| 1 | 120-122 | Process on track |
| 2 | ≤119 or ≥123 | Patient sodium possibly not as expected; duplicate measurement for confirmation |
| 3 | Confirmed/duplicated ≤119 or ≥123 | Alert on inconsistent sodium data and considerations of possible root causes:<br><br>- Check for dialysate composition<br>- Check for specific sodium inputs or losses<br>- Confirm patient fluid balance status (versus expected evolution)<br>- Interruption(s) in the RRT process |

Analysis of the sources of variation may also include other factors (e.g., expected accuracy on electrolyte distribution volume). In case of confirmed data inconsistency and failure to identify a relevant root cause among those listed, it shall be concluded that either initial sodium data was erroneous or that the electrolyte distribution volume is significantly different from predicted. It should be noted that in the case of an 'integrated' solution having continuous access to the RRT device parameters, the actual interruptions of the RRT treatment should be included in the computation of the expected patient sodium level at 6 hours.

G. RRT Controlled Correction Profile Application Embodiment

In the example where the system in embedded to the RRT machine 20 or operated on a separate device (e.g., the clinician server 106) allowing for bi-directional data transfer (including transfer of prescription parameters), the system is configured to 'continuously' adjust the RRT prescription parameters in order to change the patient electrolyte concentration according to a clinical target defined as a predefined profile/pace over time. In this application the clinical target is defined as: the target concentration to be reached at a certain point of time, and/or the concentration profile over time from current time/concentration to this target concentration (e.g. linear evolution over time).

By 'continuously', it is meant that RRT prescription parameters are automatically adjusted periodically, e.g. every 60 to 240 min, in order to keep the patient concentration profile along with the target over time. In order to achieve this, the model 122 is configured to have the option to adjust at least one of the RRT prescription parameters. If required, before initiating the process, the system could display the range of parameters planned to be used over the treatment period for validation.

This example is built considering a RRT machine 20 producing both dialysate and replacement solutions with an on-line RRT machine 20 is configured to allow for continuous changes in the sodium concentration of the fluids. The RRT machine 20 is configured as to deliver for a linear change in the patient plasma concentration with RRT prescription parameters updated every 3 hours. The table below shows adjustable fluid sodium concentration in CVVHDF.

| Patient data | | | RRT data | | |
|---|---|---|---|---|---|
| BWref (kg) | 63 | | *parameter* | *Flow rate** | *[Na] (mM)* |
| Hct (%) | 35 | | blood | 170 | N/A |
| Sodium kinetic data | | | Pre-replacement | 500 | |
| Cp0 (mM) | 124 | | Dialysate | 1000 | Adjustable |
| $\Delta$Vhydr0 (L) | 4.0 | | Post-replacement | 500 | |
| Vcorr (L) | -1.3 | | Fluid removal | 80 | N/A |
| Correction time (h) | 24 | | | | |
| $Cp_{target}$ (mM) | 132 | | | | |
| Cp profile | linear | | | | |
| *\* ml/min for blood, ml/h for other flow rates* | | | | | |

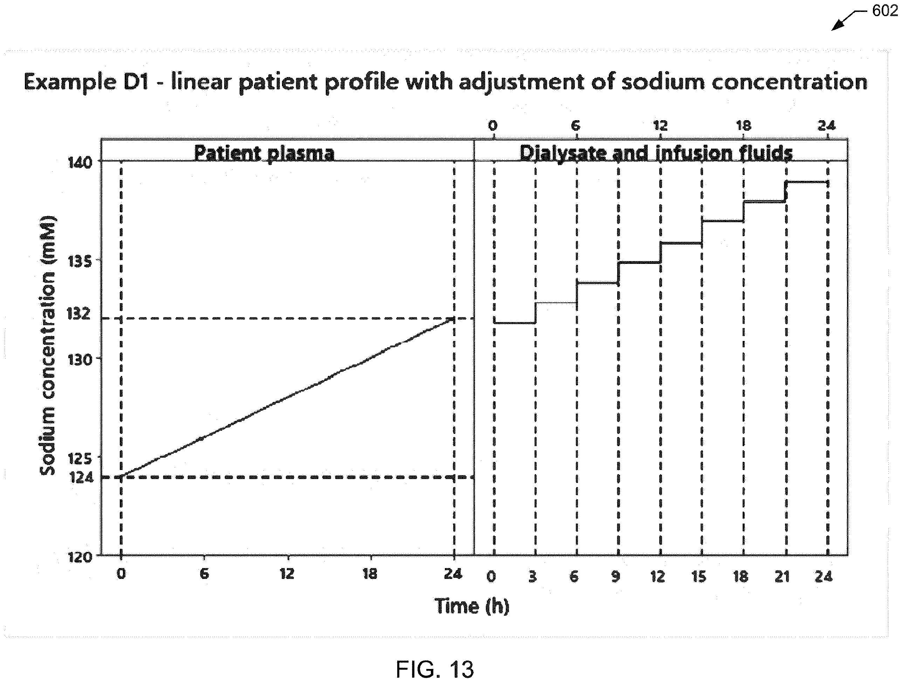

Fig. 13 shows a corresponding model output with periodic adjustment of fluids sodium concentration to deliver a linear increase of patient plasma concentration over time.

In another example, the machine 20 uses the same RRT prescription parameters as discussed above, but considers an RRT machine 20 operating with fluid bags, for which it is thus practically impossible to operate with fluid composition changes every 3 hours. Accordingly, sodium concentration of the fluid bag is to be kept constant over the correction period and the degree of freedom is provided for the flow rate. In the reported example, the ratios of pre and post-replacement flow to dialysate flow are kept identical, meaning:

$$Qpre = \frac{Qd}{2}$$

$$Qpost = \frac{Qd}{2} \quad (17)$$

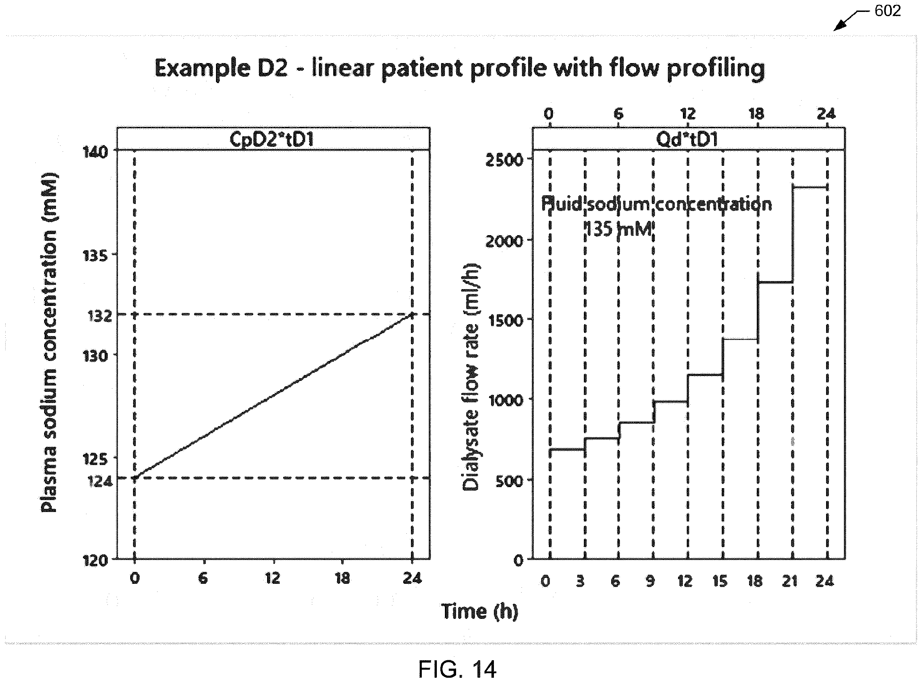

Fig. 14 shows a model output for this example with periodic adjustment of fluid flow rates to deliver a linear increase of patient plasma concentration over time. This example shows the possibility to deliver a linear change in the patient plasma sodium with fluids at fixed sodium concentration and while delivering a relevant CRRT dose. In this example, the average dialysate flow rate is 1230 ml/h, matching the delivery of a higher CRRT dose as in the reference example above. Flow rates range and average value are strongly dependent on the selected sodium concentration of the fluids and may be optimized by the RRT machine 20 and/or the clinician server 106.

### H. Electrolyte Correction with Flow Adjustment at a Constant CRRT Dose Application Embodiment

This example applies to a system that bags where it is not practically possible to make periodic changes in the sodium concentration. In this scenario, solutions of different sodium content are used to control the sodium balance of the extracorporeal blood circuit. Further, in this example, an isotonic glucose solution is used in post-dilution. Same patient parameters and sodium target as discussed above are kept and following table shows the changes made on the RRT system 20. The table below shows RRT parameters applicable to this example with changes from the above example shown in colored cells.

| RRT data - example D3 | | |
| --- | --- | --- |
| *parameter* | *Flow rate\** | *[Na] (mM)* |
| blood | 170 | N/A |
| Pre-replacement | 500 | 140 |
| Dialysate | 1500-Qpost | 140 |
| Post-replacement | Adjustable | 0 |
| Fluid removal | 80 | N/A |
| *\* ml/min for blood, ml/h for other flow rates* | | |

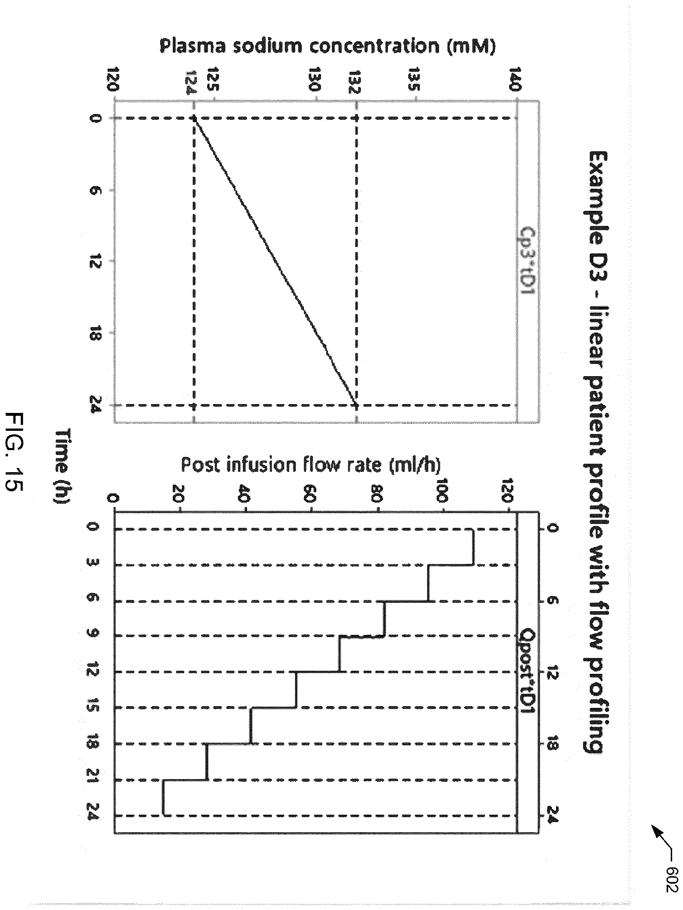

Fig. 15 shows a model output for this example with periodic adjustment of post-infusion and dialysate flow rates to deliver a linear increase of patient plasma concentration over time. This example shows the possibility to deliver a linear change in the patient plasma sodium in a system with two bags of different sodium content and while keeping the CRRT dose constant along the process (differently from the previous example). Although not shown in Fig. 15, the dialysate flow rate also varies between each 3-hour time period (from ~1390 up to ~1485 ml/h from beginning to end).

### IV. CRRT Down Time Embodiment

In some instances, RRT for a patient may have down times, which may occur along a prediction time window. Accuracy of the prediction can be improved by anticipating some of the down times. The down times may occur when a patient is moved for other medical procedures, such as radiographic imaging. Down times may also occur when bags are replaced on the RRT machine 20. Experience indicates that bag change steps match typically with about 5% down time. As these interruptions are individually short and distributed all along the delivered therapy, the effect can be considered via a corresponding decrease of the operating flow rates as to simulate a treatment dose much closer to the actual dose to be delivered. This 5% estimate is adapted in specific systems where effluent fluid may be automatically drained. Further, the rate may further be adjusted to the actual flow rates and size of bags.

Another important source of down time relates to the need to change the blood extracorporeal circuit to reduce potential for clotting. Although typically difficult to anticipate, the change of the extracorporeal circuit may be considered in specific circumstances (e.g., running RRT for some time). In this scenario, the model is configured to consider a 'long' down-time (e.g. 30 to 60 min) at some point along the prediction time window to improve accuracy.

In the circumstances where the disclosed system, methods, and apparatus have real time access to the RRT system data (e.g., the patient data 114), an occurrence of significant down time is configured to trigger a revision of the model 122. For example, the model 122 may cause an alert to be output to a clinician with an updated estimate of the patient status at the end of the prediction window. Alternatively, the model 122 is configured to generate a new recommendation for RRT prescription parameters needed to achieve a previously set target. The model 122 may further determine periodic changes to be made to the RRT prescription parameters to reach the clinical target.

It should be appreciated that other changes to patient volume or electrolyte status may occur during RRT. For example, infusion of a specific crystalloid infusion fluid may be stopped. This infusion stoppage is recorded in the EMR, either automatically, or via clinician input. The stopping of the infusion influences volume status and electrolyte concentrations of the patient going forward. When this is detected by the system disclosed herein, the model 122 is configured to update the rate of change and/or generate an alert for a clinician.

V. Conclusion

It should be understood that various changes and modifications to the presently preferred embodiments described herein will be apparent to those skilled in the art. Such changes and modifications can be made without departing from the spirit and scope of the present subject matter and without diminishing its intended advantages. It is therefore intended that such changes and modifications be covered by the appended claims.

**Claims**

1. A system for plasma electrolyte management for continuous renal replacement therapies ("RRT"), the system comprising:

   a first memory device storing patient data for a patient, the patient data including:
   patient blood data from an initial blood test, fluid status, body weight, infusion data, dialysis data, urine output, and RRT prescription parameters associated with a prescribed clinical target;
   a second memory device storing:
   a kinetic physiological model configured to calculate a current and estimated future plasma electrolyte concentration in the patient using the patient data; and
   a processor communicatively coupled to the first memory and the second memory, the processor configured to

      determine or receive a current plasma electrolyte concentration,
      calculate at least one of a electrolyte rate of change or an estimated future plasma electrolyte concentration in the patient using the kinetic physiological model taking into account the current plasma electrolyte concentration, and
      cause the at least one of the electrolyte rate of change or the estimated future plasma electrolyte concentration to be displayed.

2. The system of Claim 1, wherein the processor is further configured to:

   compare the electrolyte rate of change to a threshold, and
   when the electrolyte rate of change exceeds the threshold, generate a message indicative that at least some of the RRT prescription parameters should be changed to reduce the electrolyte rate of change or achieve the clinical target,
   in particular wherein the plasma electrolyte includes at least one of sodium, potassium, or phosphorus.

3. The system of any one of the previous Claims, wherein the processor, the first memory device, and the second memory device are located in a RRT machine, optionally the processor being remote from and in communication with a RRT machine.

   wherein the RRT machine includes at least one of a continuous RRT machine or a hemodialysis machine, optionally wherein the system is for acute RRTs.

4. The system of any one of the previous Claims, wherein the processor is further configured to:

   calculate a change to at least some of the RRT prescription parameters to reduce the electrolyte rate of change to be below the threshold; and
   apply the calculated change to the RRT prescription parameters such that the RRT machine operates according to the changed RRT prescription parameters, or cause the calculated change to the RRT prescription parameters to be displayed for clinician verification,
   in particular wherein the calculated change to the at least some of the RRT prescription parameters is configured to achieve at least one of a target equilibrium concentration of the plasma electrolyte concentration or a pace/rate of correction of the plasma electrolyte concentration.

5. The system of any one of the previous Claims, wherein the processor is further configured to:

receive additional patient data generated at any time;
use the kinetic physiological model to determine a new current plasma electrolyte concentration and a new estimated future plasma electrolyte concentration in the patient for calculating an instantaneous, new electrolyte rate of change; and
determine new RRT prescription parameters based on the new electrolyte rate of change and the new current plasma electrolyte concentration.

6. The system of any one of the previous Claims, wherein the estimated future plasma electrolyte concentration is calculated for a subsequent time period of at least one of four hours, eight hours, twelve hours, sixteen hours, 24 hours, 48 hours, or 96 hours.

7. The system of any one of the previous Claims, wherein the RRT prescription parameters includes at least one of a patient fluid removal rate, an effluent flow rate, a filter K0A, a blood flow rate, a pre-replacement fluid flow rate, a dialysate or dialysis fluid flow rate, a post-replacement fluid flow rate, a pre-replacement electrolyte concentration, a post-replacement electrolyte concentration, or a dialysate electrolyte concentration.

8. The system of any one of the previous Claims, wherein the processor is further configured to estimate the future plasma electrolyte concentration in the patient by:

using the infusion data, dialysis data, urine output, and RRT prescription parameters to estimate future water, fluid, and electrolyte input/output amounts; and
applying the estimated future water, fluid, and electrolyte input/output amounts to the kinetic physiological model.

9. The system of any one of the previous Claims, wherein the dialysis data includes electrolyte removal in effluent determined by the processor computing the current plasma electrolyte concentration using a RRT machine that is fluidly coupled to the patient.

10. The system of any one of the previous Claims, wherein the kinetic physiological model to determine the estimated future plasma electrolyte concentration in the patient includes periodic 5% to 20% per hour down times for changing bags of a RRT machine and a long down time of 30 to 180 minutes to replace an extracorporeal circuit of the RRT machine at least once during a prediction window.

11. The system of any one of the previous Claims, wherein the processor is further configured to:

receive an indication of a down time of a RRT machine;
update the kinetic physiological model based on a length of the down time; and
at least one of generate an alert message indicative of the down time, generate a message indicative of a new current plasma electrolyte concentration and a new estimated future plasma electrolyte concentration in the patient based on the down time, generate a message indicative of a new rate of change of a plasma electrolyte concentration, or generate a message indicative that the RRT prescription parameters should be changed to reach the clinical target when the RRT is resumed.

12. A system for plasma electrolyte management for continuous renal replacement therapies ("RRT"), the system comprising:

a RRT machine configured to administer a RRT to a patient according to a RRT prescription parameters associated with a prescribed clinical target;
a first memory device storing patient data for a patient, the patient data including:
patient blood data from an initial blood test, infusion data, dialysis data, and urine output;
a second memory device storing:
a kinetic physiological model configured to calculate a current and estimated future plasma electrolyte concentration in the patient using the patient data; and
a processor communicatively coupled to the RRT machine, the first memory, and the second memory, the processor configured to

use the kinetic physiological model to determine a current plasma electrolyte concentration and an estimated future plasma electrolyte concentration in the patient over a prediction window,
compare the current plasma electrolyte concentration and the estimated future plasma electrolyte concentration to the prescribed clinical target,

determine at least some new RRT prescription parameters based on the comparison to meet the prescribed clinical target, and

transmit a message to the RRT machine with the new RRT prescription parameters, the message causing the RRT machine to administer the RRT using the new RRT prescription parameters, or transmit a message to a clinician device with the new RRT prescription parameters for clinician verification.

13. The system of Claim 12, wherein the processor, the first memory device, and the second memory device are located in a RRT machine, in particular wherein the RRT machine includes at least one of a continuous RRT machine or a hemodialysis machine.

14. The system of Claims 12 or 13, wherein the processor is further configured to:

compare a predicted rate of change of a future plasma electrolyte concentration based on the new RRT prescription parameters to at least one threshold of corresponding to a maximum rate of change in plasma electrolyte concentration; and

when the threshold is exceeded, modify the RRT prescription parameters to be below the threshold,

in particular wherein the new the RRT prescription parameters are configured to achieve at least one of a target equilibrium concentration of the plasma electrolyte concentration or a pace/rate of correction of the plasma electrolyte concentration.

15. A system for plasma electrolyte management for continuous renal replacement therapies ("RRT"), the system comprising:

a first memory device storing patient data for a patient, the patient data including:

patient blood data from an initial blood test, fluid status, infusion data, body weight, an expected/prescribed patient volume correction ("$V_{corr}$") over a prediction time period, and RRT prescription parameters associated with a prescribed clinical target;

a second memory device storing:

a kinetic physiological model configured to calculate a current and estimated future plasma electrolyte concentration in the patient using the patient data; and

a processor communicatively coupled to the first memory and the second memory, the processor configured to

determine or receive a current plasma electrolyte concentration,

calculate a correction rate of the patient fluid volume ("Qnet") over the prediction time period,

calculate at least one of a electrolyte rate of change or an estimated future plasma electrolyte concentration in the patient using the kinetic physiological model taking into account the current plasma electrolyte concentration and the correction rate of the patient fluid volume ("Qnet"), and

cause the at least one of the electrolyte rate of change or the estimated future plasma electrolyte concentration to be displayed.

FIG. 1

EP 4 184 520 A1

FIG. 2

Replacement pump is pushing fluid into the blood path (pre- or post- or both)

Blood pump is creating a positive pressure pushing fluid with solutes from the blood side of the filter through the membrane

Effluent pump pulls the fluid with solutes from the blood-side of the filter through the membrane to the non-blood side

pre-replacement

pre/ post-replacement

62
64
70
60
68
66
76b
72
76a
20
74

EP 4 184 520 A1

FIG. 3

INFUSION FLUID/
WATER/
ELECTROLYTE
INPUTS-OUTPUTS

RRT FLUID/WATER/
ELECTROLYTE
INPUTS-OUTPUTS

URINE FLUID/
WATER/
ELECTROLYTE
INPUTS-OUTPUTS

BLOOD TEST DATA

RRT PRESCRIPTION

PROCESSOR/
SERVER

CURRENT PLASMA
ELECTRODE
CONCENTRATION

PREDICTED PLASMA
ELECTRODE
CONCENTRATION

RATE OF CHANGE

PACE/RATE
CORRECTION

FIG. 4

PROCESSOR (40) CLINICIAN
SERVER (106)

500

START

114

RECEIVE PATIENT DATA — 502

PREDICT FLUID, WATER, ELECTROLYTE INPUTS/
OUTPUTS — 504

122

APPLY A KINETIC PHYSIOLOGICAL MODEL TO THE
PREDICTED FLUID, WATER, ELECTROLYTE INPUTS/
OUTPUTS — 506

OUTPUT A CURRENT AND/OR FURTHER PLASMA
ELECTROLYTE CONCENTRATION — 508

DETERMINE A RATE OF CHANGE — 510

IS CORRECTION NEEDED? — 512
NO

YES

DETERMINE A RATE OF CORRECTION — 514

APPLY THE RATE OF CORRECTION — 402
516

FIG. 5

FIG. 6

FIG. 7

Predicted patient plasma sodium kinetics

Plasma sodium concentration (mM)

Cp = 146.0 mM

24 hour change 11.0 mM

Cp*t

Time (h)

Daily plasma concentration change rate (mmol/L/day)

Desired max rate

dCpday*t

602

**FIG. 8**

602

Predicted patient sodium concentration kinetics

Plasma sodium concentration (mM)

Cp=146.0 mM

Cp=150.5 mM

CpA2*tA2

simulation
1
2

Daily plasma concentration change rate (mM/day)

dCpA2*tA2

Time (h)

EP 4 184 520 A1

FIG. 9

Predicted patient plasma sodium kinetics

FIG. 10

Output from example B2

Dialysate sodium (mM)

Maximum daily plasma sodium change rate (mM/day)

Dialysate flow rate (ml/h)

FIG. 11

602

Predicted patient plasma sodium kinetics

FIG. 12

Example D1 - linear patient profile with adjustment of sodium concentration

FIG. 13

EP 4 184 520 A1

FIG. 14

Example D3 - linear patient profile with flow profiling

FIG. 15

602

Europäisches
Patentamt

European
Patent Office

Office européen
des brevets

# EUROPEAN SEARCH REPORT

**Application Number**

**EP 21 30 6604**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/257891 A1 (AKONUR ALP [US] ET AL) 20 October 2011 (2011-10-20) * paragraph [0059] – paragraph [0232] * | 1-15 | INV. G16H20/40 |
| A | Chiari Lorenzo ET AL: "Prediction of solute kinetics, acid- base status, and blood volume changes during profiled hemodialysis Cite this paper A Crit ical Review of Sodium Profiling for Hemodialysis", , 1 January 2000 (2000-01-01), pages 204-215, XP55912937, Retrieved from the Internet: URL:https://www.academia.edu/download/50709508/1.24520161204-5157-1cgq3mm.pdf [retrieved on 2022-04-14] * the whole document * | 1-15 | |

**TECHNICAL FIELDS SEARCHED (IPC)**

G16H

The present search report has been drawn up for all claims

| Place of search | Date of completion of the search | Examiner |
|---|---|---|
| Munich | 25 April 2022 | Rinelli, Pietro |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
...............................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 21 30 6604

This annex lists the patent family members relating to the patent documents cited in the above-mentioned European search report.
The members are as contained in the European Patent Office EDP file on
The European Patent Office is in no way liable for these particulars which are merely given for the purpose of information.

25-04-2022

| Patent document cited in search report | Publication date | Patent family member(s) | Publication date |
|---|---|---|---|
| US 2011257891 A1 | 20-10-2011 | US 2011257891 A1 | 20-10-2011 |
| | | US 2012029324 A1 | 02-02-2012 |
| | | US 2015032043 A1 | 29-01-2015 |
| | | US 2017182234 A1 | 29-06-2017 |
| | | US 2019351121 A1 | 21-11-2019 |

For more details about this annex : see Official Journal of the European Patent Office, No. 12/82